# EUROPEAN PATENT SPECIFICATION

(11) **EP 4 305 201 B1**
(45) Date of publication and mention of the grant of the patent: **09.09.2026**
(21) Application number: 22766349.9
(22) Date of filing: 10.03.2022
(51) Int. Cl.: C12Q 1/6869, C12Q 1/6841

(54) **CHEMICAL SAMPLE INDEXING FOR HIGH-THROUGHPUT SINGLE-CELL ANALYSIS**
CHEMISCHE PROBENINDIZIERUNG ZUR EINZELZELLANALYSE MIT HOHEM DURCHSATZ
INDEXATION CHIMIQUE DES ÉCHANTILLONS POUR L'ANALYSE UNICELLULAIRE À HAUT DÉBIT

(30) Priority: 10.03.2021 WO PCT/CN2021/079903
(43) Date of publication of application: 17.01.2024
(73) Proprietor: Nanjing University, Nanjing, Jiangsu 210023 (CN); Singleron (Nanjing) Biotechnologies, Ltd., Nanjing, Jiangsu 211800 (CN)
(72) Inventor: LI, Jie, Nanjing, Jiangsu 210023 (CN); ZHAO, Xinlu, Nanjing, Jiangsu 210023 (CN); YU, Wenhao, Nanjing, Jiangsu 210023 (CN); YANG, Yang, Nanjing, Jiangsu 210023 (CN); ZHU, Wenqi, Nanjing, Jiangsu 211800 (CN); SUN, Dafei, Nanjing, Jiangsu 211800 (CN); DING, Xiuheng, Nanjing, Jiangsu 211800 (CN)
(74) Representative: Witte, Weller und Partner Patentanwälte mbB Stuttgart
(86) International application number: PCT/CN2022/080093
(87) International publication number: WO 2022/188827

(56) References cited:
- WO-A1-2018/170515
- WO-A1-2019/113499
- CN-A- 111 378 728
- GB-A- 2 582 850
- US-A1- 2018 346 969
- MARLON STOECKIUS ET AL: "Cell Hashing with barcoded antibodies enables multiplexing and doublet detection for single cell genomics", GENOME BIOLOGY, vol. 19, no. 1, 1 December 2018 (2018-12-01), XP055702284, DOI: 10.1186/s13059-018-1603-1
- GENTILE STEFAN D. ET AL: "Click Chemistry-Based DNA Labeling of Cells for Barcoding Applications", BIOCONJUGATE CHEMISTRY, vol. 29, no. 8, 22 July 2018 (2018-07-22), US, pages 2846 - 2854, XP093231937, ISSN: 1043-1802, DOI: 10.1021/acs.bioconjchem.8b00435
- ZHAO XINLU ET AL: "Improved ClickTags enable live-cell barcoding for highly multiplexed single cell sequencing", RSC CHEMICAL BIOLOGY, vol. 3, no. 8, 1 January 2022 (2022-01-01), pages 1052 - 1060, XP093220540, ISSN: 2633-0679, DOI: 10.1039/D2CB00046F
- MCGINNIS CHRISTOPHER S.; PATTERSON DAVID M.; WINKLER JULIANE; CONRAD DANIEL N.; HEIN MARCO Y.; SRIVASTAVA VASUDHA; HU JENNIFER L.;: "MULTI-seq: sample multiplexing for single-cell RNA sequencing using lipid-tagged indices", NATURE METHODS, vol. 16, no. 7, 17 June 2019 (2019-06-17), New York, pages 619 - 626, XP036901129, ISSN: 1548-7091, DOI: 10.1038/s41592-019-0433-8
- GUO CHUNER, KONG WENJUN, KAMIMOTO KENJI, RIVERA-GONZALEZ GUILLERMO C., YANG XUE, KIRITA YUHEI, MORRIS SAMANTHA A.: "CellTag Indexing: genetic barcode-based sample multiplexing for single-cell genomics", GENOME BIOLOGY, vol. 20, no. 1, 1 December 2019 (2019-12-01), pages 1 - 13, XP055964684, DOI: 10.1186/s13059-019-1699-y

## Description

### REFERENCE TO SEQUENCE LISTING

The present application is being filed along with a Sequence Listing in electronic format. The Sequence Listing is provided as a file entitled Sequence _Listing_76PP-328958-WO, created March 7, 2022, which is 4 kilobytes in size.

### TECHNICAL FIELD/BACKGROUND

The present disclosure relates generally to the field of molecular biology, specifically methods and compositions for sample indexing for high-throughput single-cell analysis.

### BACKGROUND

Single-cell and single-nucleus RNA sequencing (scRNA-seq and snRNA-seq) have become powerful techniques for studying heterogeneous transcription profiles in multicellular systems. With the advent of single-cell sequencing technologies, parallel transcriptional analysis of 10³-10⁵ cells or nuclei is now routine. This improves the prospects for screening of hundreds or even thousands of samples for high-throughput analysis of genetic, signal, and drug perturbations.

While next generation sequencing and library construction cost has been significantly reduced, routine analysis of thousands of cells is still expensive for individual laboratories. There are still a wide range of challenges, including reliable recognition of artifacts caused by cell multiplets or technology-dependent batch effects. It has been proven that in the integrated analysis of scRNA-seq experiments, technology and "batch" effects can mask biological signals, so experimental solutions are needed to alleviate these challenges.

Sample multiplexing methods such as CITE-seq, MULTI-seq, or Cell Tag Indexing addressed the issues by labeling cells with sample-specific barcodes before pooling and single cell separation. In these technologies, sample-specific barcodes just as transcripts are linked to cell barcodes during reverse transcription, such that cells are divided into sample groups by tracking cells with same sample-specific barcodes.

In a "Cell-Hashing" method, a known oligonucleotide sequence is conjugated with an antibody, which is then used to label a cell via an antigen-antibody specific reaction between the antibody and a cell surface protein. However, the price of antibodies is relatively expensive, which leads to higher costs. At the same time, the antigen-antibody reaction depends on the specific cell surface protein for labeling. For various types of clinical samples, it is impossible to label all types of cells, and thus causes certain cell types in the sample to be "lost." Another method to label cells is called "Click-Tag," in which methyltetrazine-modified DNA oligonucleotides are connected to cell proteins through an inverse-electron demand Diels-Alder (IEDDA) reaction with heterobifunctional, amine-reactive crosslinker NHS-trans-cyclooctene (NHS-TCO). However, this method is limited to labeling fixed cells, not living cells. It is known that cell fixation can cause cell damage, leading to changes in gene expression in cells.

WO 2018/170515 discloses spatially resolved single-cell analysis methods using barcoded probes to study cellular phenotypes and interactions within tissues.

WO 2019/113499 describes high-throughput combinatorial perturbation approaches (e.g., CRISPR-based) with single-cell readouts for identifying gene interactions and reconstructing regulatory networks.

Gentile et al. Bioconjugate Chem. 2018, 29, 2846-2854, report a click-chemistry-based method for labeling cells with DNA oligonucleotide barcodes attached to the cell surface, enabling multiplexed tracking, sorting, and quantitative analysis of cell populations via flow cytometry and PCR.

There remains a need for efficient cell labeling and sample indexing methods for high-throughput analysis of various types of cells.

### SUMMARY

Provided herein include methods, reagents, compositions, systems and kits for cell labeling and high-throughput single-cell nucleic acid analysis.

The present invention relates to a method of indexing a plurality of samples. The method comprises: (a) providing a plurality of samples, wherein each of the plurality of samples comprises a plurality of cells, wherein the plurality of cells comprises living cells and is derived from cell separation; (b) for each of the plurality of samples, contacting a coupling agent with the sample, wherein the coupling agent comprises a coupling group and a first reactive group, thereby associating the coupling agent to the surface of the plurality of cells; and (c) for each of the plurality of samples, contacting a plurality of indexing labels each comprising an identical sample-specific-indexing sequence and a second reactive group capable of forming a covalent bond with the first reactive group of the coupling agent in an inverse electron demand Diels-Alder (IEDDA) reaction, thereby generating a plurality of cells each associated with the sample-specific-indexing label, wherein the sample-specific-indexing sequence for each sample is different from other samples.

The present invention also relates to a method of analyzing nucleic acids. The method comprises: (a) providing a plurality of samples wherein each of the plurality of samples comprises a plurality of cells, wherein the plurality of cells comprises living cells and is derived from cell separation; (b) for each of the plurality of samples, contacting a coupling agent with the sample, wherein the coupling agent comprises a coupling group and a first reactive group, thereby associating the coupling agent to the surface of the plurality of cells; (c) for each of the plurality of samples, contacting a plurality of indexing labels each comprising an identical sample-specific-indexing sequence and a second reactive group capable of forming a covalent bond with the first reactive group of the coupling agent in an inverse electron demand Diels-Alder (IEDDA) reaction, thereby generating a plurality of cells each associated with the sample-specific-indexing label, wherein the sample-specific-indexing sequence for each sample is different from other samples; (d) pooling the plurality of cells associated with the sample-specific-indexing labels from the plurality of samples to form a pooled sample with a plurality of pooled cells; (e) partitioning the plurality of pooled cells into a plurality of partitions, thereby at least 25% of the plurality of partitions each comprises a single cell of the plurality of pooled cells; (f) analyzing target nucleic acids associated with the single cell, wherein the target nucleic acids comprise the indexing labels associated with the single cell, and (g) determining the sample origin of single cell based on the sample-specific-indexing sequence associated with the single cell.

In some embodiments of the method disclosed herein, at least least 75% of the plurality of partitions each comprises a single cell of the plurality of pooled cells.

In some embodiments, analyzing target nucleic acids comprises sequencing the target nucleic acids, products thereof (e.g., amplification products thereof), or a portion of the target nucleic acids or products thereof. In some embodiments, determining the sample origin of single cell comprises high temperature denaturation, fragment sorting, or a combination thereof.

In some embodiments, the target nucleic acids comprise cellular nucleic acids, viral nucleic acids, bacterial nucleic acids, mitochondrial nucleic acids, synthetic nucleic acids, or amplification product thereof, or a combination thereof. In some embodiments, the target nucleic acids comprise deoxyribonucleic acid (DNA), ribonucleic acid (RNA), or a combination thereof. In some embodiments, the target nucleic acids comprise poly-adenylated messenger ribonucleic acid (mRNAs) of the single cell.

In some embodiments, analyzing target nucleic acids comprises barcoding in the plurality of partitions comprising a single cell, using a plurality of barcode molecules in a single partition, to generate barcoded target nucleic acids. In some embodiments, barcoding the target nucleic acids comprises barcoding (i) the indexing labels associated with the single cell and (ii) mRNAs of the single cells to generate (i-a) a barcoded indexing label and (ii-a) barcoded cDNAs. In some embodiments, barcoding target nucleic acids comprises a reverse transcription reaction, and the barcoded targeted nucleic acids comprises complementary deoxyribonucleic acid (cDNA). In some embodiments, a barcode molecule of the plurality of barcode molecules comprises a cell barcode sequence, a molecular label sequence, a primer sequence (e.g., a sequencing primer sequence), a primer binding site, a template switching oligonucleotide, or a combination thereof. In some embodiments, the barcode molecules of the plurality of barcode molecules in a single partition comprise an identical cell barde sequence and different molecular label sequence. In some embodiments, the molecular label sequences comprise unique molecule identifiers (UMIs).

The length of the molecular label sequence can vary, for example 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, or more, or any number or any range between two of these values. In some embodiments, the molecular label sequence is or is about 2-40 nucleotides in length. The sequencing primer sequence can be, for example, a Read 1 sequence, a Read 2 sequence, or a portion thereof.

In some embodiments, in each of the plurality comprising a single cell, the plurality of barcode molecules are attached to, reversibly attached to, covalently attached to, or irreversibly attached to a bead. In some embodiments, barcoding in a single partition comprises partitioning the bead into the single partition. In some embodiments, partitioning the plurality of pooled cells into a plurality of partitions comprises co-partitioning the pooled cells and the bead into the single partitions. The bead can be, for example, a solid bead. In some embodiments, the bead is a magnetic bead or a polymer bead.

In some embodiments, the plurality of partitions comprises droplets or microwells. In some embodiments, analyzing target nucleic acids comprises introducing a plurality of template switching oligonucleotides into the partition and barcoding the plurality of target nucleic acids by extending the plurality of barcode molecules using the target nucleic acids and the plurality of template switching oligonucleotides as templates to generate barcoded nucleic acids.

In some embodiments, analyzing target nucleic acids comprises introducing a plurality of extension primers to the partition and barcoding the target nucleic acids by extending the plurality of extension primers using the target nucleic acids as templates and the plurality of barcode molecules as template switching oligonucleotides to generate barcoded nucleic acids.

In some embodiments, the coupling group of the coupling agent is capable of forming a covalent bond with the surface of the plurality of cells. In some embodiments, the coupling agent and/or the indexing label further comprises a hydrophilic group, for example a hydrophilic group comprises PEG. In some embodiments, the coupling agent is NHS-PEG4-TCO. In some embodiments, coupling group of the coupling agent is capable of forming a covalent bond with -NH2 on the surface of the plurality of cells. The plurality of cells can comprise fixed cells, living cells, or any combination thereof.

In some embodiments, each of the indexing labels is a 5' NHS-PEG5-Tz modified oligonucleotide. In some embodiments, each of the indexing labels further comprises a PCR handle sequence, a UMI, a capture sequence, or a combination thereof. In some embodiments, the capture sequence comprises a poly(dA) sequence. In some embodiments, the indexing labels are single-stranded DNA. In some embodiments, first reactive group and the second reactive group form the second covalent bond in an inverse electron demand Diels-Alder (IEDDA) reaction. In some embodiments, one of the first reactive group and the second reactive group comprises a tetrazine (Tz) group and the other comprises a trans-cyclooctene (TCO) group.

The number of the samples in the plurality of samples can vary. For example, the plurality of samples can comprise, comprise at least, or comprise at most, 3, 6, 12, 15, 18, 21, 24, 27, 30, 40, 50, 60, 70, 80, 90, 100, 150, 200, 250, 300, 350, 400, 450, 500, 600, or a number or a range between any two of these values, samples. In some embodiments, the plurality of samples comprise at least 12 samples. The samples can be, for example clinical samples, environmental samples, biological samples, or a combination thereof. In some embodiments, the plurality of cells comprises prokaryotic cells, eukaryotic cells, or a combination thereof.

The method can, for example, comprise washing the plurality of cells before step (a) and/or resuspending the cells in aqueous solution. In some embodiments, the method comprises washing the plurality of cells to remove unbound coupling agent after step (b) and before step (c) and/or resuspending the cells in aqueous solution. In some embodiments, the method comprises washing the cells associated with the sample-specific-indexing labels to remove unbound sample-specific-indexing labels after step (c) and/or resuspending the cells in aqueous solution.

Also disclosed herein include a kit for indexing a plurality of samples. The kit can comprise a coupling agent comprising a coupling group and a first reactive group; for each of the plurality of samples, a plurality of indexing labels each comprising an identical sample-specific-indexing sequence and a second reactive group capable of forming a covalent bond with the first reactive group of the coupling agent; and instructions to use the kit for indexing multiple samples according to any one of the methods disclosed herein for indexing multiple samples.

Also disclosed herein include a kit for analyzing nucleic acids in a plurality of samples. The kit can comprises a coupling agent each comprising a coupling group and a first reactive group; for each of the plurality of samples, a plurality of indexing labels each comprising an identical sample-specific-indexing sequence and a second reactive group capable of forming a covalent bond with the first reactive group of the coupling agent; a plurality of beads, wherein each bead is attached to, reversibly attached to, covalently attached to, or irreversibly attached to a plurality of barcode molecules, and wherein each barcode molecule of the plurality of barcode molecules comprises a cell barcode sequence, a molecular label sequence, a primer sequence, a primer binding site, a template switching oligonucleotide, or a combination thereof; and instructions to use the kit for indexing multiple samples according to any one of the methods disclosed herein for indexing multiple samples.

The present disclosure provides a method for cell labeling for high-throughput single-cell RNA sequencing. The method can comprise:
a) labeling a living cell through chemically linked index;
b) pooling up to 12 samples in one experiment;
c) reverse transcription and sample index extension in one step;
d) sample index separation and library construction; and
e) analyzing sample index library.

In some aspects, the cell labeling comprises reaction of the cell with a chemical reagent.

In some aspects, the index sequence can additionally comprise a poly(A) sequence that can be capture with oligo-dT.

In some aspects, the sample index can comprise a unique molecular index (UMI) sequence that can be used to quantification and data split.

In some aspects, the sample index can be separated by high temperature denaturation.

In some aspects, the sample index can be separated by fragment sorting.

In some aspects, the sample index library is analyzed by sequencing.

The present disclosure also provides the chemical reagents used in the present method. The present disclosure also provides a product that includes reagents needed to carry out the present method of cell labeling.

### BRIEF DESCRIPTION OF DRAWINGS

FIG. 1 shows a schematic illustration of a non-limiting exemplary flow chart of the method of present disclosure.
FIG. 2 shows quality control result (size in bp) of a representative Tag amplified library.
FIG. 3 shows mass spectrometry of S'NHS-PEG5-Tz modified sample-specific barcodes.
FIG. 4A-FIG. 4B show t-distributed stochastic neighbor embedding (t-SNE) plots colored by cluster and by Tag.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

Provided herein include methods, reagents, compositions, systems and kits for cell labeling and high-throughput single-cell nucleic acid analysis. The methods, reagents, compositions, systems, and kits can be used for sample indexing to allow high-throughput single-cell analysis (e.g., single-cell RNA sequencing), and can also be used for fast and convenient cell labeling and sample pooling. The present methods provide an efficient way to label cells and index samples, and can have broad applicability in cell labeling without being limited by cell types. Combined with single-cell sequencing, the methods disclosed herein can greatly reduce the cost of single cell analysis, and simultaneously improve the throughput of sample processing. In addition, the present sample indexing method can be used to facilitate the wide application of high-throughput single cell transcriptome sequencing.

### Method of Sample Indexing

Disclosed herein include methods of indexing multiple samples comprising cells. The method according to the invention comprises: (a) providing a plurality of samples, wherein each of the plurality of samples comprises a plurality of cells, wherein the plurality of cells comprises living cells and is derived from cell separation; (b) for each of the plurality of samples, contacting a coupling agent with the sample, wherein the coupling agent comprises a coupling group and a first reactive group, thereby associating the coupling agent to the surface of the plurality of cells; and (c) for each of the plurality of samples, contacting a plurality of indexing labels each comprising an identical sample-specific-indexing sequence and a second reactive group capable of forming a covalent bond with the first reactive group of the coupling agent in an inverse electron demand Diels-Adler (IEDDA) reaction, thereby generating a plurality of cells each associated with the sample-specific-indexing label, wherein the sample-specific-indexing sequence for each sample is different from other samples. The characteristics of the cells to which the method can be applied to are not particularly limited. The state, type, or morphology of the cells can vary. For example, the cells can be living cells, fixed cells, healthy cells, cells in disease state, or any combination thereof.

The association of the coupling agent to the cell surface can be based on, or a result of, the covalent bond. The association of the coupling agent and/or the label to the cell surface can be independent of, or unaffected by, the cell type or any specific cell surface proteins (such as antibodies, antigens, structural proteins, or receptors). Accordingly, a wide variety of cells can be labeled using the present methods.

### Coupling agent

The surface of the cell can comprise a surface reactive group that reacts with the coupling group of the coupling agent to form a covalent bond. The surface reactive group and the coupling group can include, but are not limited to, known crosslinking groups and their counterparts. For example, the surface reactive group and the coupling group can be selected from nucleophilic groups, such as an amino group (-NH₂), a hydroxy group (-OH), a sulfhydryl group (-SH), or a carboxylate group (-COOH), and corresponding electrophilic groups, such as activated carboxylate groups, including but not limited to acid chlorides, anhydrides, carbodiimide derivatives, and N-hydroxysuccinimide (NHS) esters. In some aspects, the surface reactive group is an amino group (e.g., -NH₂ on a side chain of an amino acid of a surface protein) and the coupling group is an NHS group. Other suitable coupling pairs for the surface reactive group and the coupling group can be selected according to known technologies. In some aspects, the cell surface can be modified to introduce the surface reactive group prior to the attachment of the coupling agent.

After attachment of the coupling agent to the cell surface, the first reactive group of the coupling agent can be exposed (e.g., toward the outside of the cell) for reaction with the second reactive group of the label, thereby attaching the label to the cell surface through the coupling agent. In some aspects, the first reactive group and the second reactive group form the second covalent bond in an inverse electron demand Diels-Alder (IEDDA) reaction. The IEDDA cycloaddition as a click chemistry conjugation reaction has been used for a variety of applications, including labeling of nanoparticles, antibodies, oligonucleotides, small molecules, and radiopharmaceuticals. For example, one of the first and the second reactive group can comprise a tetrazine (Tz) group, and the other can comprise a trans-cyclooctene (TCO) group. In some aspects, the first reactive group comprises a TCO group and the second reactive group comprises a Tz group.

The coupling agent can comprise a hydrophilic group. The presence of the hydrophilic group can improve the aqueous solubility of the coupling agent to facilitate cell labeling. Suitable hydrophilic groups can include for example, hydrophilic polymers such as polyethylene glycol (PEG), poly(2-oxazoline), poly(vinyl alcohol), or polyacrylate. In some aspects, the hydrophilic group comprises PEG. The PEG group can include one or more ethylene glycol (-CH₂CH₂O-) units. For example, the hydrophilic groups can comprise, comprise about, comprise at least, comprise at least about, comprise at most, or comprise at most about, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any two of these values, ethylene glycol units. In some aspects, the hydrophilic group comprises 3, 4, 5, 6, 7, or 8 ethylene glycol units. In some aspects, the hydrophilic group comprises 4 or 5 ethylene glycol units. In some aspects, the coupling agent is NHS-PEG4-TCO.

### Indexing label

The indexing label can comprise an identical sample-specific-indexing sequence and a reactive group capable of forming a covalent bond with the reactive group of the coupling agent. In some aspects, the reactive group of the indexing label can be a function group (e.g., Tz or TCO) that forms a covalent bond with the reactive group of the coupling agent, for example in an inverse electron demand Diels-Alder (IEDDA) reaction as described herein. The indexing label can be referred to herein as indexing oligonucleotide, sample index oligo, sample-specific barcode, or sample tag.

The indexing label can comprise a sample-specific-indexing sequence (also referred to herein as sample barcode sequence). The sample-specific-indexing sequence can be used to identify a sample which the cell being labeled is from. The sample can be, for example, a cell line, a biological sample, an environmental sample, or a forensic sample.

A sample can comprise one or more types of cells. The number of types of cells in a sample can be different in different aspects. The sample can comprise, comprise about, comprise at least, comprise at least about, comprise at most, or comprise at most about, 1, 2, 3, 4, 5, 6, 7, 8, 9, 10, 20, 30, 40, 50, 60, 70, 80, 90, 100, 1000, 10000, or a number or a range between any two of these values, types of cells. In some aspects, the sample comprises one type of cells. In some aspects, the sample is a cell line.

The number of cells in a sample to be labeled can be different in different aspects. The number of cells in different samples of the plurality of samples to be labeled can also be different in different aspects. The number of cells in a sample can be, be about, be at least, be at least about, be at most, or be at most about, 100, 1000, 1x10⁴, 1x10⁵, 1x10⁶, 1x10⁷, 1x10⁸, 1x10⁹, 1x10¹⁰, or a number or a range between any two of these values. Cells of a sample can be identified by an identical sample-specific-indexing sequence. In some aspectss, at least two cells from a same sample are labeled with identical sample-specific-indexing sequences. Cells of different samples can be labeled with different sample-specific-indexing sequences. In some aspects, at least two cells from different samples are labeled with different sample-specific-indexing sequences.

The sample-specific-indexing sequence can be, be about, be at least, be at least about, be at most, or be at most about, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, or a number or a range between any two of these values, nucleotides in length.

The indexing label can further comprise, for example, a PCR handle sequence, a random index sequence (e.g., an unique molecular index, or UMI), and/or a capture sequence (e.g., a poly(A) primer sequence). The random index sequence can be used to identify the molecular origin of the indexing labels and/or quantify the abundance of indexing labels on the labeled cell.

In some aspects, the indexing label further comprises a poly(A) sequence, such as a poly(A) tail. An indexing label comprising a poly(A) sequence can be reverse transcribed, for example, by a reverse transcriptase.

The indexing label can be a single stranded DNA (ssDNA). In some aspects, the indexing label is, is about, is at least, is at least about, is at most, or is at most about, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values, nucleotides in length. In some aspects, the indexing label comprises or consists of a sequence of SEQ ID NO:1.

The indexing label can further comprise a hydrophilic group. The presence of the hydrophilic group can improve the aqueous solubility of the label to facilitate cell labeling. Suitable hydrophilic groups can include for example, hydrophilic polymers such as polyethylene glycol (PEG), poly(2-oxazoline), poly(vinyl alcohol), or polyacrylate. In some aspects, the hydrophilic groups comprise PEG. The PEG group can include one or more ethylene glycol (-CH₂CH₂O-) units. For example, the hydrophilic groups can comprise, comprise about, comprise at least, comprise at least about, comprise at most, or comprise at most about, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any two of these values, ethylene glycol units. In some aspects, the hydrophilic group comprises 3, 4, 5, 6, 7, or 8 ethylene glycol units. In some aspects, the hydrophilic group comprises 4 or 5 ethylene glycol units.

The indexing label can be prepared, for example, by attaching the indexing label to the reactive group through a coupling reaction. In some aspects, the label is prepared by reacting an indexing label having a terminal amino group (e.g., 5' modified NH2-C6-ssDNA) with an NHS activated molecule comprising the second reactive group (e.g., NHS-PEG5-Tz).

### Labeling the cell

The cell (e.g., a living cell) can be treated with the coupling agent to generate the activated cell surface, and the label comprising the indexing label is subsequently attached to the activated cell surface (e.g., in an IEDDA reaction). In some aspects, the cell having the activated cell surface is washed to remove the unbound coupling agent before attaching the label. In some aspects, the cell is a living cell, and reacting the living cell with the coupling agent and the label can be carried out in a one-pot reaction. After labeling, the cells (e.g., living cells) can be washed and counted.

One or more types of cells, or cells from one or more samples, can be labeled using the present method. For example, different types of cells, or cells from different samples, can be labeled with labels having different sample-specific-indexing sequences according to the labeling methods disclosed herein.

A plurality of cells (e.g., living cells) labeled with labels having different sample-specific-indexing sequences can be pooled to generate pooled labeled cells for further analysis. The number of different sample-specific-indexing sequences in the pooled labeled cells can be, be about, be at least, be at least about, be at most, or be at most about, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any two of these values.

### Method of Analyzing Nucleic Acid

Disclosed herein include methods of analyzing nucleic acids. In some aspects, the method comprises a method of analyzing nucleic acids. The method according to the invention comprises: (a) providing a plurality of samples wherein each of the plurality of samples comprises a plurality of cells, wherein the plurality of cells comprises living cells and is derived from cell separation; (b) for each of the plurality of samples, contacting a coupling agent with the sample, wherein the coupling agent comprises a coupling group and a first reactive group, thereby associating the coupling agent to the surface of the plurality of cells; (c) for each of the plurality of samples, contacting a plurality of indexing labels each comprising an identical sample-specific-indexing sequence and a second reactive group capable of forming a covalent bond with the first reactive group of the coupling agent in an inverse electron demand Diels-Adler (IEDDA) reaction, thereby generating a plurality of cells each associated with the sample-specific-indexing label, wherein the sample-specific-indexing sequence for each sample is different from other samples; (d) pooling the plurality of cells associated with the sample-specific-indexing labels from the plurality of samples to form a pooled sample with a plurality of pooled cells; (e) partitioning the plurality of pooled cells into a plurality of partitions, thereby at least 25% of the plurality of partitions each comprises a single cell of the plurality of pooled cells; (f) analyzing target nucleic acids associated with the single cell, wherein the target nucleic acids comprise the indexing labels associated with the single cell, and (g) determining the sample origin of single cell based on the sample-specific-indexing sequence associated with the single cell.

In some aspects of the method, at least 30%, at least 35%, at least 40%, at least 45%, at least 50%, at least 55%, at least 60%, at least 65%, at least 70%, at least 75%, at least 80%, at least 85%, at least 90%, at least 95%, at least 98%, at least 99%, or at least 100% of the plurality of partitions each comprises a single cell of the plurality of pooled cells.

Analyzing target nucleic acids can, for example, comprise sequencing the target nucleic acids, products thereof (e.g., amplification products thereof), or a portion of the target nucleic acids or products thereof. In some aspects, determining the sample origin of single cell comprises high temperature denaturation, fragment sorting, or a combination thereof. Target nucleic acids can, for example, comprise cellular nucleic acids, viral nucleic acids, bacterial nucleic acids, mitochondrial nucleic acids, synthetic nucleic acids, or amplification product thereof, or a combination thereof. In some aspects, the target nucleic acids comprise deoxyribonucleic acid (DNA), ribonucleic acid (RNA), or a combination thereof. In some aspects, the target nucleic acids comprise poly-adenylated messenger ribonucleic acid (mRNAs) of the single cell.

The cells (e.g., living cells) can be labeled, for example, using the labeling method as described herein. In some aspectss, the indexing label comprises a sample-specific-indexing sequence and a random index sequence. In some aspects, the indexing label further comprises a poly(A) sequence, such as a poly(A) tail. In some aspects, the indexing label is a single-stranded DNA.

In some aspects, the analyzing step of the present method comprises barcoding (i) the indexing label of the label of the plurality of labels covalently bonded to the single cell or single living cell and (ii) the plurality of target nucleic acids associated with the single cell or single live cell using a plurality of barcode molecules to generate (i-a) a barcoded indexing label and (ii-a) a plurality of barcoded nucleic acids. In some aspects, the method further comprise analyzing (e.g., sequencing) the barcoded indexing label and the plurality of barcoded nucleic acids.

In some aspects, the cells (e.g., living cells) are labeled with indexing labels each comprising a poly(A) sequence, such that target nucleic acids (e.g., RNAs) associated with the cells and the indexing label attached to the cells can be barcoded (e.g., by reverse transcription) and analyzed simultaneously.

The present disclosure provides an efficient and cost-effective cell labeling method, which can be used for multiplexed cellular analysis, including, for example, multiplexed single-cell RNA sequencing (scRNA-seq).

In some easpects, the present methods includes labeling a cell surface by reacting NHS-reactive amines on the cell surface with NHS-PEG4-TCO, followed by an inverse electron-demand Diels-Alder (IEDDA) reaction to attach 5'NHS-PEG5-Tz modified sample-specific barcodes to the NHS-PEG4-TCO on the cell surface. The sample-specific oligo can include a PCR handle sequence, a sample barcode sequence, a UMI sequence (for molecular quantification), and/or a poly(A) tail. In some aspects, the oligo label on the cell surface can be captured and reverse transcribed and amplified just like mRNA molecules. After sequencing, sample barcode mapping and quantification can be used to split the mixed sample sequencing data, thereby achieving scRNA-seq sample demultiplexing. The present disclosure can employ chemical modification methods to label living cells and can have a wide range of applicability.

### Partition

A partition as used herein refers to a part, a portion, or a division sequestered from the rest of the parts, portions, or divisions. A partition can be formed through the use of wells, microwells, multi-well plates, microwell arrays, microfluidics, dilution, dispensing, droplets, or any other means of sequestering one fraction of a sample from another. In some aspects, a partition is a well, a droplet or a microwell. In some aspects, a partition is a microwell.

The plurality of partitions can comprise at least 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, or 500000 partitions. In some aspectss, the plurality of partitions comprises at least 100 partitions.

The barcode molecules can be introduced to the partitions directly. The barcode molecules can be attached to a particle (e.g., a bead), and introducing the barcode molecules can comprise introducing the particle to a partition. In some aspects, barcode molecules can be introduced into the partitions (e.g., microwells) by attaching or synthesizing the plurality of barcode molecules onto the surface of the partitions.

The plurality of partitions can comprise a plurality of microwells of a microwell array. The microwell array can comprise different numbers of microwells in different implementations. In some aspects, the microwell array can comprise, comprise about, comprise at least, comprise at least about, comprise at most, or comprise at most about, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, or a number or a range between any two of these values, microwells. The microwells can be arranged into rows and columns. The number of microwells in a row (or a column) can be, be about, be at least, be at least about, be at most, or be at most about, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, or a number or a range between any two of these values. Adjacent rows (or columns) of microwells can be aligned or staggered, for example.

The width, length, depth (or height), radius, or diameter of a microwell can vary. In some aspects, the width, length, depth (or height), radius, or diameter of a microwell of the plurality of microwells can be, be about, be at least, be at least about, be at most, or be at most about, 1 µm, 2 µm, 3 µm, 4 µm, 5 µm, 6 µm, 7 µm, 8 µm, 9 µm, 10 µm, 20 µm, 30 µm, 40 µm, 50 µm, 60 µm, 70 µm, 80 µm, 90 µm, 100 µm, 110 µm, 120 µm, 130 µm, 140 µm, 150 µm, 160 µm, 170 µm, 180 µm, 190 µm, 200 µm, 210 µm, 220 µm, 230 µm, 240 µm, 250 µm, 260 µm, 270 µm, 280 µm, 290 µm, 300 µm, 310 µm, 320 µm, 330 µm, 340 µm, 350 µm, 360 µm, 370 µm, 380 µm, 390 µm, 400 µm, 410 µm, 420 µm, 430 µm, 440 µm, 450 µm, 460 µm, 470 µm, 480 µm, 490 µm, 500 µm, 600 µm, 700 µm, 800 µm, 900 µm, 1000 µm, or a number or a range between any two of these values. For example, the width, the length, and/or depth of a microwell can be 10 µm to 200 µm, including 10, 20, 30, 40, 50, 60, 70, 80, 100, 110, 120, 130, 140, 150, 160, 170, 180, 190, or 200 µm, or a number or a range between any two of these values. The shape of a microwell can vary, for example circular, elliptical, square, rectangular, triangular, or hexagonal shape.

The volume of one, one or more, or each, of the plurality of microwells can be different in different aspects. The volume of one, one or more, or each, of the plurality of microwells can be, be about, be at least, be at least about, be at most, or be at most about, 1 nm³, 2 nm³, 3 nm³, 4 nm³, 5 nm³, 6 nm³, 7 nm³, 8 nm³, 9 nm³, 10 nm³, 20 nm³, 30 nm³, 40 nm³, 50 nm³, 60 nm³, 70 nm³, 80 nm³, 90 nm³, 100 nm³, 200 nm³, 300 nm³, 400 nm³, 500 nm³, 600 nm³, 700 nm³, 800 nm³, 900 µm³, 1000 nm³, 10000 nm³, 100000 µm³, 1000000 nm³, 10000000 nm³, 100000000 µm³, 1000000000 nm³, 2 µm³, 3 µm³, 4 µm³, 5 µm³, 6 µm³, 7 µm³, 8 µm³, 9 µm³, 10 µm³, 20 µm³, 30 µm³, 40 µm³, 50 µm³, 60 µm³, 70 µm³, 80 µm³, 90 µm³, 100 µm³, 200 µm³, 300 µm³, 400 µm³, 500 µm³, 600 µm³, 700 µm³, 800 µm³, 900 µm³, 1000 µm³, 10000 µm³, or a number or a range between any two of these values. The volume of one, one or more, or each, of the plurality of microwells can be, be about, be at least, be at least about, be at most, or be at most about, 1 nanolieter (nl), 2 nl, 3 nl, 4 nl, 5 nl, 6 nl, 7 nl, 8 nl, 9 nl, 10 nl, 11 nl, 12 nl, 13 nl, 14 nl, 15 nl, 16 nl, 17 nl, 18 nl, 19 nl, 20 nl, 21 nl, 22 nl, 23 nl, 24 nl, 25 nl, 26 nl, 27 nl, 28 nl, 29 nl, 30 nl, 31 nl, 32 nl, 33 nl, 34 nl, 35 nl, 36 nl, 37 nl, 38 nl, 39 nl, 40 nl, 41 nl, 42 nl, 43 nl, 44 nl, 45 nl, 46 nl, 47 nl, 48 nl, 49 nl, 50 nl, 51 nl, 52 nl, 53 nl, 54 nl, 55 nl, 56 nl, 57 nl, 58 nl, 59 nl, 60 nl, 61 nl, 62 nl, 63 nl, 64 nl, 65 nl, 66 nl, 67 nl, 68 nl, 69 nl, 70 nl, 71 nl, 72 nl, 73 nl, 74 nl, 75 nl, 76 nl, 77 nl, 78 nl, 79 nl, 80 nl, 81 nl, 82 nl, 83 nl, 84 nl, 85 nl, 86 nl, 87 nl, 88 nl, 89 nl, 90 nl, 91 nl, 92 nl, 93 nl, 94 nl, 95 nl, 96 nl, 97 nl, 98 nl, 99 nl, 100 nl, or a number or a range between any two of these values. For example, the volume of one, one or more, or each, of the plurality of microwells is about 1 nm³ to about 10000 µm³.

The microwell array comprising a plurality of microwells can be formed from any suitable material. In some aspects, a microwell array comprising a plurality of microwells can be formed from a material selected from silicon, glass, ceramic, elastomers such as polydimethylsiloxane (PDMS) and thermoset polyester, thermoplastic polymers such as polystyrene, polycarbonate, poly(methyl methacrylate) (PMMA), poly-ethylene glycol diacrylate (PEGDA), Teflon, polyurethane (PU), composite materials such as cyclic-olefin copolymer, and combinations thereof.

Partitions (e.g., microwells or droplets) can be introduced with samples, free reagents, and/or reagents encapsulated in microcapsules. The reagents can comprise restriction enzymes, ligase, polymerase, fluorophores, oligonucleotide barcodes, oligonucleotide probes, adapters, buffers, dNTPs, ddNTPs, and other reagents required for performing the methods described herein.

Cells (e.g., cells labeled using the labeling methods disclosed herein) and/or particles (e.g., beads, including barcoding beads disclosed herein) can be partitioned (separately partitioned or co-partitioned) into a plurality of partitions. As a result of partitioning, the percentage of the plurality of partitions comprising a desired number of cell(s) (e.g., a single cell), a single particle (e.g., a bead) or both of a desired number of cell(s) and a single particle can vary. For example, the percentage of the plurality of partitions comprising the desired number of cell(s) and/or a single particle can be, be about, be at least, be at least about, be at most, or be at most about, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or a number or a range between any two of these values. In some aspects, at least 10%, at least 25%, at least 50%, at least 75%, or at least 90% of the plurality of partitions comprise a desired number of cell(s). In some aspects, at least 10%, at least 25%, at least 50%, at least 75%, or at least 90% of the plurality of partitions comprise a desired number of cell(s) and a single particle.

The percentage of the plurality of partitions comprising no cell can vary. For example, the percentage of the plurality of partitions comprising no cell can be, be about, be at least, be at least about, be at most, or be at most about, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, or a number or a range between any two of these values. In some aspects, at most 50% of partitions of the plurality of partitions can comprise no cell of the plurality of cells.

The percentage of the plurality of partitions comprising more than the desired number of cell(s) can be different in different aspects. For example, the percentage of the plurality of partitions comprising more than the desired number of cell(s) can be, be about, be at least, be at least about, be at most, or be at most about, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, or a number or a range between any two of these values. In some aspects, at most 10% of partitions of the plurality of partitions can comprise more than the desired number of cell(s).

### Target Nucleic Acids

As described herein, cells can be associated with target nucleic acids. For example, a cell can comprise one or more target nucleic acids (e.g., mRNA) or can be labeled with one or more target nucleic acids (e.g., directly, or indirectly through a binding moiety, such as an antibody conjugated with the nucleic acid). The target nucleic acids associated with the cell can be from, on the surface of, or binding to the surface of the cell. A target nucleic acid can have a sequence (e.g., an mRNA sequence, excluding the poly(A) tail).

The target nucleic acids associated with the cell can comprise deoxyribonucleic acid (DNA), ribonucleic acid (RNA), and/or any combination or hybrid thereof. The target nucleic acids can be single-stranded or double-stranded, or contain portions of both double-stranded or single-stranded sequences. The target nucleic acids can contain any combination of nucleotides, including uracil, adenine, thymine, cytosine, guanine, inosine, xanthine, hypoxanthine, isocytosine, isoguanine and any nucleotide derivative thereof. As used herein, the term "nucleotide" can include naturally occurring nucleotides and nucleotide analogs, including both synthetic and naturally occurring species. The target nucleic acids can be genomic DNA (gDNA), mitochondrial DNA (mtDNA), messenger RNA (mRNA), ribosomal RNA (rRNA), transfer RNA (tRNA), nuclear RNA (nRNA), small interfering RNA (siRNA), small nuclear RNA (snRNA), small nucleolar RNA (snoRNA), small Cajal body-specific RNA (scaRNA), microRNA (miRNA), double stranded (dsRNA), ribozyme, riboswitch or viral RNA, or any nucleic acids that may be obtained from a sample.

The plurality of target nucleic acids can, for example, comprise DNA, gDNA, RNA, and/or mRNA. In some aspects, the plurality of target nucleic acids comprises mRNA, for example a poly-adenylated mRNA.

### Barcoding

In some aspects of the methods disclosed herein, analyzing target nucleic acids comprises barcoding in the plurality of partitions comprising a single cell, using a plurality of barcode molecules in a single partition, to generate barcoded target nucleic acids. In some aspects, barcoding the target nucleic acids comprises barcoding (i) the indexing labels associated with the single cell and (ii) mRNAs of the single cells to generate (i-a) a barcoded indexing label and (ii-a) barcoded cDNAs. In some aspects, barcoding target nucleic acids comprises a reverse transcription reaction, and the barcoded targeted nucleic acids comprises cDNA. In some aspects, a barcode molecule of the plurality of barcode molecules comprises a cell barcode sequence, a molecular label sequence, a primer sequence (e.g., a sequencing primer sequence), a primer binding site, a template switching oligonucleotide, or a combination thereof. In some aspects, the barcode molecules of the plurality of barcode molecules in a single partition comprise an identical cell barde sequence and different molecular label sequence. In some aspects, the molecular label sequences comprise UMIs.

In the methods disclosed herein, barcode molecules can be introduced into the partitions for barcoding indexing labels and target nucleic acids. For example, the amount of the barcode molecules added directly to a partition can be, be about, be at least, be at least about, be at most, or be at most about, 0.1 ng, 0.2 ng, 0.3 ng, 0.4 ng, 0.5 ng, 0.6 ng, 0.7 ng, 0.8 ng, 0.9 ng, 1 ng, 2 ng, 3 ng, 4 ng, 5 ng, 6 ng, 7 ng, 8 ng, 9 ng, 10 ng, 20 ng, 30 ng, 40 ng, 50 ng, 60 ng, 70 ng, 80 ng, 90 ng, 100 ng, 200 ng, 300 ng, 400 ng, 500 ng, 600 ng, 700 ng, 800 ng, 900 ng, 1000 ng, 2000 ng, 3000 ng, 4000 ng, 5000 ng, 6000 ng, 7000 ng, 8000 ng, 9000 ng, 10000 ng, 2000 ng, 3000 ng, 4000 ng, 5000 ng, 6000 ng, 7000 ng, 8000 ng, 90000 ng or a number or a range between any two of these values.

The barcode molecules introduced into the partitions (e.g., microwells or droplets) can be associated with particles (e.g., beads). In some aspects, introducing the plurality of barcode molecules to the partition comprises introducing a particle comprising the plurality of barcode molecules to the partition. The particles can provide a surface upon which molecules, such as oligonucleotides, can be synthesized or attached. In some aspects, the plurality of barcode molecules are attached to, reversibly attached to, covalently attached to, or irreversibly attached to the particle.

The particle can comprise, comprise about, comprise at least, comprise at least about, comprise at most, or comprise at most about, 10, 50, 100, 1000, 10000, 20000, 30000, 40000, 50000, 60000, 70000, 80000, 90000, 100000, 200,000, 300,000, 400,000, 500,000, 600,000, 700,000, 800,000, 900,000, 1000000, 2000000, 3000000, 4000000, 5000000, 6000000, 7000000, 8000000, 9000000, 10000000, 20000000, 50000000, 100000000, or a number or a range between any two of these values, barcode molecules. The attachment of barcode molecules to the particle can be covalent or non-covalent via non-covalent bonds such as ionic bonds, hydrogen bonds, or van der Waals interactions. The attachment can be direct to the surface of a particle or indirect through other oligonucleotide sequences attached to the surface of a particle.

The particle (e.g., a bead) can be dissolvable, degradable, or disruptable. A particle can be a gel particle such as a hydrogel particle. In some aspects, the gel particle is degradable upon application of a stimulus. The stimulus can comprise a thermal stimulus, a chemical stimulus, a biological stimulus, a photo-stimulus, or a combination thereof. The particle can be a solid particle and/or a magnetic particle. In some aspects, the particle is a magnetic particle. The magnetic particle can comprise a paramagnetic material coated or embedded in the magnetic particle (e.g. on a surface, in an intermediate layer, and/or mixed with other materials of the magnetic particle). A paramagnetic material refers to a material having a magnetic susceptibility slightly greater than 1 (e.g. between about 1 and about 5). A magnetic susceptibility is a measure of how much a material can become magnetized in an applied magnetic field. Paramagnetic materials include, but not limited to, magnesium, molybdenum, lithium, aluminum, nickel, tantalum, titanium, iron oxide, gold, copper, or a combination thereof. In some aspects, the magnetic particle comprising barcode molecules can be immobilized or retained in a partition (such as a microwell or a well) by an external magnetic field, thereby retaining the barcode molecules in a partition. The magnetic particle comprising barcode molecules can be mobilized or released when the external magnetic field is removed.

A particle can, for example, be immobilized or retained in a partition (e.g., a microwell) through an interaction between two members of a binding pair. For example, the partition (e.g., microwell) can be coated with a capture moiety (e.g., a member of a binding pair) capable of binding with a binding moiety (the other member of the binding pair) comprised in or conjugated to a particle, such that the binding of the two moieties results in the attachment of the particle to the partition, thereby immobilizing or retaining the particle in the partition. For example, the surface of a partition can be coated with streptavidin. The biotinylated particle can be attached to the surface of the partition via streptavidin-biotin interaction.

Particles can be of uniform size or heterogeneous size. For example, one or more of the particles can have a diameter of about, at least, at least about, at most, or at most about, 1 µm, 5 µm, 10 µm, 20 µm, 30 µm, 40 µm, 45 µm, 50 µm, 60 µm, 65 µm, 70 µm, 75 µm, 80 µm, 90 µm, 100 µm, 250 µm, 500 µm, or 1 mm.

In some aspects, a particle can be sized such that at most one particle, not two particles, can fit one partition. A size or dimension (e.g., length, width, depth, radius, or diameter) of a particle can be different in different aspects. For example, a size or dimension of one, or each, particle can be, be about, be at least, be at least about, be at most, or be at most about, 20 nanometer (nm), 21 nm, 22 nm, 23 nm, 24 nm, 25 nm, 26 nm, 27 nm, 28 nm, 29 nm, 30 nm, 31 nm, 32 nm, 33 nm, 34 nm, 35 nm, 36 nm, 37 nm, 38 nm, 39 nm, 40 nm, 41 nm, 42 nm, 43 nm, 44 nm, 45 nm, 46 nm, 47 nm, 48 nm, 49 nm, 50 nm, 51 nm, 52 nm, 53 nm, 54 nm, 55 nm, 56 nm, 57 nm, 58 nm, 59 nm, 60 nm, 61 nm, 62 nm, 63 nm, 64 nm, 65 nm, 66 nm, 67 nm, 68 nm, 69 nm, 70 nm, 71 nm, 72 nm, 73 nm, 74 nm, 75 nm, 76 nm, 77 nm, 78 nm, 79 nm, 80 nm, 81 nm, 82 nm, 83 nm, 84 nm, 85 nm, 86 nm, 87 nm, 88 nm, 89 nm, 90 nm, 91 nm, 92 nm, 93 nm, 94 nm, 95 nm, 96 nm, 97 nm, 98 nm, 99 nm, 100 nm, 110 nm, 120 nm, 130 nm, 140 nm, 150 nm, 160 nm, 170 nm, 180 nm, 190 nm, 200 nm, 210 nm, 220 nm, 230 nm, 240 nm, 250 nm, 260 nm, 270 nm, 280 nm, 290 nm, 300 nm, 310 nm, 320 nm, 330 nm, 340 nm, 350 nm, 360 nm, 370 nm, 380 nm, 390 nm, 400 nm, 410 nm, 420 nm, 430 nm, 440 nm, 450 nm, 460 nm, 470 nm, 480 nm, 490 nm, 500 nm, 510 nm, 520 nm, 530 nm, 540 nm, 550 nm, 560 nm, 570 nm, 580 nm, 590 nm, 600 nm, 610 nm, 620 nm, 630 nm, 640 nm, 650 nm, 660 nm, 670 nm, 680 nm, 690 nm, 700 nm, 710 nm, 720 nm, 730 nm, 740 nm, 750 nm, 760 nm, 770 nm, 780 nm, 790 nm, 800 nm, 810 nm, 820 nm, 830 nm, 840 nm, 850 nm, 860 nm, 870 nm, 880 nm, 890 nm, 900 nm, 910 nm, 920 nm, 930 nm, 940 nm, 950 nm, 960 nm, 970 nm, 980 nm, 990 nm, 1000 nm, 2 micrometer (µm), 3 µm, 4 µm, 5 µm, 6 µm, 7 µm, 8 µm, 9 µm, 10 µm, 20 µm, 30 µm, 40 µm, 50 µm, 60 µm, 70 µm, 80 µm, 90 µm, 100 µm, 110 µm, 120 µm, 130 µm, 140 µm, 150 µm, 160 µm, 170 µm, 180 µm, 190 µm, 200 µm, 210 µm, 220 µm, 230 µm, 240 µm, 250 µm, 260 µm, 270 µm, 280 µm, 290 µm, 300 µm, 310 µm, 320 µm, 330 µm, 340 µm, 350 µm, 360 µm, 370 µm, 380 µm, 390 µm, 400 µm, 410 µm, 420 µm, 430 µm, 440 µm, 450 µm, 460 µm, 470 µm, 480 µm, 490 µm, 500 µm, or a number or a range between any two of these values. In some aspects, a size or dimension of one, or each, particle is about 1 nm to about 100 µm. In some aspects, the particle can have a dimension about 10 µm to about 100 µm (e.g., 30 µm).

The volume of one, or each, particle can vary. The volume of one, or each, particle can be, be about, be at least, be at least about, be at most, or be at most about, 1 nm³, 2 nm³, 3 nm³, 4 nm³, 5 nm³, 6 nm³, 7 nm³, 8 nm³, 9 nm³, 10 nm³, 20 nm³, 30 nm³, 40 nm³, 50 nm³, 60 nm³, 70 nm³, 80 nm³, 90 nm³, 100 nm³, 200 nm³, 300 nm³, 400 nm³, 500 nm³, 600 nm³, 700 nm³, 800 nm³, 900 µm³, 1000 nm³, 10000 nm³, 100000 µm³, 1000000 nm³, 10000000 nm³, 100000000 µm³, 1000000000 nm³, 2 µm³, 3 µm³, 4 µm³, 5 µm³, 6 µm³, 7 µm³, 8 µm³, 9 µm³, 10 µm³, 20 µm³, 30 µm³, 40 µm³, 50 µm³, 60 µm³, 70 µm³, 80 µm³, 90 µm³, 100 µm³, 200 µm³, 300 µm³, 400 µm³, 500 µm³, 600 µm³, 700 µm³, 800 µm³, 900 µm³, 1000 µm³, 10000 µm³, 100000 µm³, 1000000 µm³, or a number or a range between any two of these values. The volume of one, or each, particle can be, be about, be at least, be at least about, be at most, or be at most about, 1 nanoliter (nL), 2 nL, 3 nL, 4 nL, 5 nL, 6 nL, 7 nL, 8 nL, 9 nL, 10 nL, 11 nL, 12 nL, 13 nL, 14 nL, 15 nL, 16 nL, 17 nL, 18 nL, 19 nL, 20 nL, 21 nL, 22 nL, 23 nL, 24 nL, 25 nL, 26 nL, 27 nL, 28 nL, 29 nL, 30 nL, 31 nL, 32 nL, 33 nL, 34 nL, 35 nL, 36 nL, 37 nL, 38 nL, 39 nL, 40 nL, 41 nL, 42 nL, 43 nL, 44 nL, 45 nL, 46 nL, 47 nL, 48 nL, 49 nL, 50 nL, 51 nL, 52 nL, 53 nL, 54 nL, 55 nL, 56 nL, 57 nL, 58 nL, 59 nL, 60 nL, 61 nL, 62 nL, 63 nL, 64 nL, 65 nL, 66 nL, 67 nL, 68 nL, 69 nL, 70 nL, 71 nL, 72 nL, 73 nL, 74 nL, 75 nL, 76 nL, 77 nL, 78 nL, 79 nL, 80 nL, 81 nL, 82 nL, 83 nL, 84 nL, 85 nL, 86 nL, 87 nL, 88 nL, 89 nL, 90 nL, 91 nL, 92 nL, 93 nL, 94 nL, 95 nL, 96 nL, 97 nL, 98 nL, 99 nL, 100 nL, or a number or a range between any two of these values. In some aspects, the volume of one, or each, particle is about 1 nm³ to about 1000000 µm³.

The number of particles introduced into a plurality of partitions can be different in different aspects. In some aspects, the number of particles introduced into a plurality of partitions is, is about, is at least, is at least about, is at most, or is at most, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 6000000, 7000000, 8000000, 9000000, 10000000, or a number or a range between any two of these values.

In some aspects, particles are introduced to the partitions such that the percentage of partitions each occupied with one particle is, is about, is at least, is at least about, is at most, or is at most about, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or a number or a range between any two of these values. In some aspects, at least 80% of the plurality of partitions is each occupied with one particle. In some aspects, particles are introduced to the partitions such that the percentage of partitions with no particle is, is about, is at least, is at least about, is at most, or is at most about, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, or a number or a range between any two of these values. In some aspects, at most 20% of the plurality of partitions contain no particle.

### Barcode Molecules

Barcode molecules (e.g., barcode molecules attached to particles) can be partitioned, for example, in microwells or wells. The term "barcode" as used herein can be a verb or a noun. When used as a noun, the term "barcode" or "barcode molecule" refers to a label that can be attached to a polynucleotide, or any variant thereof, to convey information about the polynucleotide. For example, a barcode can be a polynucleotide sequence attached to fragments of the target nucleic acids associated with a cell in the partition. The barcode can then be sequenced alone or with the fragments of the target nucleic acids associated with the cell. The presence of the same barcode on multiple sequences or different barcodes on different sequences can provide information about the cell origin and/or the molecular origin of the sequences. When used as a verb, the term "barcode" refers to a process of attaching a barcode or a barcode molecule to a target nucleic acid associated with the cell.

Barcode molecules can be generated from a variety of different formats, including pre-designed polynucleotide barcodes, randomly synthesized barcode sequences, microar-ray-based barcode synthesis, random N-mers, or combinations thereof as will be understood by a person skilled in the art.

The plurality of barcode molecules can comprise, comprise about, comprise at least, comprise at least about, comprise at most, or comprise at most about 1, 5, 10, 50, 100, 1000, 10000, 20000, 30000, 40000, 50000, 60000, 70000, 80000, 90000, 100000, 200,000, 300,000, 400,000, 500,000, 600,000, 700,000, 800,000, 900,000, 1000000, 2000000, 3000000, 4000000, 5000000, 6000000, 7000000, 8000000, 9000000, 10000000, 20000000, 50000000, 100000000, or a number or a range between any two of these values.

A barcode molecule (or a segment of a barcode molecule, such as a cell barcode sequence or a molecular barcode sequence) can be in any suitable length. For example, a barcode molecule (or a segment of a barcode molecule) can be about 2 to about 500 nucleotides in length, about 2 to about 100 nucleotides in length, about 2 to about 50 nucleotides in length, about 2 to about 40 nucleotides in length, about 4 to about 20 nucleotides in length, or about 6 to 16 nucleotides in length. In some aspects, the barcode molecule (or a segment of a barcode molecule) can be, be about, be at least, be at least about, be at most, or be at most about 2, 3, 4, 5, 6, 7, 8, 9, 10, 11, 12, 13, 14, 15, 16, 17, 18, 19, 20, 25, 30, 35, 40, 45, 50, 55, 60, 65, 70, 85, 90, 95, 100, 150, 200, 250, 300, 400, or 500 nucleotides in length, or a number or a range between any two of these values.

The barcode molecules used herein can comprise a cell barcode sequence and a molecular barcode sequence (e.g., a UMI). A barcode molecule can also comprise other sequences, such as a target binding sequence or region capable of hybridizing to target nucleic acids (e.g. poly(dT) sequence), other recognition or binding sequences, a template switching oligonucleotide (e.g., GGG, such as rGrGrG), and primer sequences (e.g. sequencing primer sequence, such as Read 1 or a PCR primer sequence) for subsequent processing (e.g. PCR amplification) and/or sequencing.

The configuration of the various sequences comprised in a barcode molecule (e.g. cell barcode sequence, UMI, primer sequence, target binding sequence or region, and/or any additional sequences) can vary depending on, for example, the particular configuration desired and/or the order in which the various components of the sequence are added as will be understood to a person skilled in the art. In some aspects, a barcode molecule has a configuration of 5'-primer sequence-cell barcode sequence-UMI-target binding sequence-3'. In some aspects, a barcode molecule has a configuration of 5'-primer sequence-cell barcode sequence-UMI-template switching oligonucleotide-3'.

### Cell barcode sequence

In some aspects, the barcode molecules can comprise a cell barcode sequence. Cell barcode sequences can be used to identify the barcoded indexing label and the barcoded nucleic acids originate from the cell. Barcoded nucleic acids that originate from the same cell (or the same partition) can have an identical cell barcode sequence. A cell barcode sequence can be referred to as a partition specific barcode, such as a microwell specific barcode. The cell barcode sequence of the barcode molecules in a partition can be identical or different.

In some aspects, the cell barcode sequences can serve to track the target nucleic acids associated with the cell throughout the processing (e.g., location of the cells in a plurality of partitions, such as microwells) when the cell barcode sequence associated with the target nucleic acids is determined during sequencing.

The number (or percentage) of barcode molecules introduced in a partition with cell barcode sequences having an identical sequence can be different in different aspects. In some aspects, the number of barcode molecules introduced in a partition with cell barcode sequences having an identical sequence is, is about, is at least, is at least about, is at most, or is at most about, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 6000000, 7000000, 8000000, 9000000, 10000000, 20000000, 30000000, 40000000, 50000000, 60000000, 70000000, 80000000, 90000000, 100000000, or a number or a range between any two of these values. In some aspects, the percentage of barcode molecules introduced in a partition with cell barcode sequences having an identical sequence is, is about, is at least, is at least about, is at most, or is at most about, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.9%, 100%, or a number or a range between any two of these values. For example, the cell barcode sequences of at least two barcode molecules introduced in a partition comprise an identical sequence. In some aspects, at least two of the cell barcode sequences of the plurality of barcode molecules in the same partition are identical.

A cell barcode sequence can be unique (or substantially unique) to a partition. The number of unique cell barcode sequences can be different in different aspects. In some aspects, the number of unique cell barcode sequences is, is about, is at least, is at least about, is at most, or is at most about, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 6000000, 7000000, 8000000, 9000000, 10000000, 20000000, 30000000, 40000000, 50000000, 60000000, 70000000, 80000000, 90000000, 100000000, or a number or a range between any two of these values. In some aspectss, the percentage of unique cell barcode sequences is, is about, is at least, is at least about, is at most, or is at most about, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.9%, 100%, or a number or a range between any two of these values, of the cell barcode sequences of the barcode molecules introduced in a partition. For example, the cell barcode sequences of barcode molecules introduced in two partitions can comprise different sequences. In some aspects, the cell barcode sequences of at least one barcode molecules in at least two different partitions are different.

In some aspectss, barcode molecules are introduced to the plurality of partitions such that different sets of a plurality of barcode molecules introduced in different partitions have different cell barcode sequences and a same set of plurality of barcode molecules introduced in a same partition have same cell barcode sequence. For example, target nucleic acids associated with a cell in a partition will be barcoded with the same cell barcode sequences.

The length of a cell barcode sequence of a barcode molecule (or a cell barcode sequence of each barcode molecule or all cell barcode sequences of the plurality of barcode molecules) can be different in different aspects. In some aspects, a cell barcode sequence of a barcode molecule (or each cell barcode sequence of each barcode molecule or all cell barcode sequences of the plurality of barcode molecules) is, is about, is at least, is at least about, is at most, or is at most about, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values, nucleotides in length.

### Molecular barcode sequence

A barcode can, for example, comprise a molecular barcode sequence or molecular label. Molecular barcode sequences can be UMIs. Molecular barcode sequences can be used to identify molecular origins of the barcoded indexing label and the barcoded nucleic acids. Molecular barcode sequences (e.g., UMIs) are short sequences used to uniquely tag each molecule in a sample in some aspects. The molecular barcode sequences of the barcode molecules partitioned into a partition can be identical or different.

In some aspects, the molecular barcode sequences of the plurality of barcode molecules are different. The number (or percentage) of molecular barcode sequences of barcode molecules introduced in a partition (e.g., a microwell or a well) with different sequences can be different in different aspectss. In some aspects, the number of molecular barcode sequences of barcode molecules introduced in a partition with different sequences is, is about, is at least, is at least about, is at most, or is at most about, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 6000000, 7000000, 8000000, 9000000, 10000000, 20000000, 30000000, 40000000, 50000000, 60000000, 70000000, 80000000, 90000000, 100000000, or a number or a range between any two of these values. In some aspects, the percentage of molecular barcode sequences of barcode molecules introduced in a partition with different sequences is, is about, is at least, is at least about, is at most, or is at most about, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.9%, 100%, or a number or a range between any two of these values. For example, the molecular barcode sequences of two barcode molecules of the plurality of barcode molecules introduced in a partition can comprise different sequences.

The number of barcode molecules introduced in a partition with molecular barcode sequences having an identical sequence can be different in different aspects. In some aspects, the number of barcode molecules introduced in a partition with molecular barcode sequences having an identical sequence is, is about, is at least, is at least about, is at most, or is at most about, 1, 2, 3, 4, 5, 6, 7, 8, 9, 10, 20, 30, 40, 50, 60, 70, 80, 90, 100, or a number or a range between any two of these values. For example, the molecular barcode sequences of two barcode molecules introduced in a partition can comprise an identical sequence.

The number of unique molecular barcode sequences can vary. For example, the number of unique molecular barcode sequences can be, be about, be at least, be at least about, be at most, or be at most about, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 6000000, 7000000, 8000000, 9000000, 10000000, 20000000, 30000000, 40000000, 50000000, 60000000, 70000000, 80000000, 90000000, 100000000, or a number or a range between any two of these values.

In some aspects, at least two of the molecular barcode sequences of the plurality of barcode molecules in a partition comprise different molecular barcode sequences (e.g., unique molecular identifiers).

The length of a molecular barcode sequence of a barcode molecule (or a molecular barcode sequence of each barcode molecule) can be different in different aspects. In some aspects, a molecular barcode sequence of a barcode molecule (or a molecular barcode sequence of each barcode molecule) is, is about, is at least, is at least about, is at most, or is at most about, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values, nucleotides in length.

In some aspects, a barcode molecule comprises a primer sequence. The primer sequence can be a sequencing primer sequence (or a sequencing primer binding sequence) or a PCR primer sequence (or PCR primer binding sequence). For example, the sequencing primer can be a Read 1 sequence. In some aspects, the barcode molecule comprises a PCR primer binding sequence, which allows for PCR amplification of a barcoded nucleic acid.

The length of the primer sequence can vary, for example the primer sequence can be 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values, nucleotides in length. The number (or percentage) of barcode molecules in a partition (e.g., a microwell) each comprising a primer sequence (or each comprising an identical primer sequence) can be different in different aspects. In some aspects, the number of barcode molecules in a partition (e.g., a microwell) each comprising a primer sequence (such as a PCR primer binding sequence) is, is about, is at least, is at least about, is at most, or is at most about, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 6000000, 7000000, 8000000, 9000000, 10000000, 20000000, 30000000, 40000000, 50000000, 60000000, 70000000, 80000000, 90000000, 100000000, or a number or a range between any two of these values. In some aspects, the percentage of barcode molecules in a partition (e.g., a microwell) each comprising a primer sequence (or each comprising an identical primer sequence) is, is about, is at least, is at least about, is at most, or is at most about, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.9%, 100%, or a number or a range between any two of these values.

In some aspects, each of the plurality of barcode molecules comprises a primer sequence (e.g., a sequencing primer sequence, including but not limited to, a Read 1 sequence, a Read 2 sequence, or a portion thereof.

In some aspects, a barcode molecule comprises a target binding sequence or region capable of hybridizing to the target nucleic acids, a particular type of target nucleic acids (e.g. mRNA), and/or specific target nucleic acids (e.g. specific gene of interest). In some aspects, the target binding sequence comprises a poly(dT) sequence and/or a sequence capable of hybridizing to the plurality of target nucleic acids.

The length of a target binding sequence can vary. For example, the target binding sequence can be, be about, be at least, be at least about, be at most, or be at most about, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values, nucleotides in length. The target binding sequence can be 12-18 deoxythymidines in length. In some aspects, the target binding sequence can be 20 nucleotides or longer to enable their annealing in reverse transcription reactions at higher temperatures as will be understood by a person of skill in the art.

In some aspects, barcode molecules comprising target binding sequences are introduced into the partitions together with other reagents such as the reverse transcription reagents. The number of the barcode molecules introduced into a partition comprising a target binding sequence can vary. For example, the number of barcode molecules introduced into a partition comprising a target binding sequence (e.g., poly(dT) sequence) can be, be about, be at least, be at least about, be at most, or be at most about, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 6000000, 7000000, 8000000, 9000000, 10000000, 20000000, 30000000, 40000000, 50000000, 60000000, 70000000, 80000000, 90000000, 100000000, or a number or a range between any two of these values.

In some aspects, the target binding sequence can be on a 3' end of a barcode molecule of the plurality of barcode molecules introduced in a partition. Barcode molecules each comprising a poly(dT) target binding sequence can be used to capture (e.g., hybridize to) a poly(A) sequence in an indexing label and/or 3' end of polyadenylated mRNA transcripts in a target nucleic acid for a downstream 3' gene expression library construction.

In some aspects, the target binding sequence comprises a poly(dT) sequence which is a single-stranded sequence of deoxythymidine (dT) used for first-strand cDNA synthesis catalyzed by reverse transcriptase. In some aspects, the target binding sequence comprises a poly(dT) sequence is introduced into the partitions as extension primers to synthesize the first-strand cDNA using the target nucleic acid (e.g. RNA) as a template.

In some aspects, the poly(dT) of the barcode molecules introduced into a partition are identical (e.g., same number of dTs). In some aspects, the poly(dT) of the barcode molecules introduced into a partition are different (e.g. different numbers of dTs). The percentage of the barcode molecules of the plurality of barcode molecules introduced into a partition with an identical poly(dT) sequence can vary. In some aspects, the percentage of the barcode molecules of the plurality of barcode molecules introduced into a partition with an identical poly(dT) sequence is, is about, is at least, is at least about, is at most, is at most about, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.9%, 100%, or a number or a range between any two of these values.

In some aspects, the target binding regions of all barcode molecules of the plurality of barcode molecules comprise poly(dT) capable of hybridizing to a poly(A) sequence in an indexing label and/or poly(A) tails of mRNA molecules (or poly(dA) regions or tails of DNA). In some aspects, the target binding regions of some barcode molecules of the plurality of barcode molecules comprise gene-specific or target-specific primer sequences. For example, a barcode molecule of the plurality of barcode molecules can also comprise a target binding region capable of hybridizing to a specific target nucleic acid associated with the cell, thereby capturing specific targets or analytes of interest. For example, the target binding region capable of hybridizing to a specific target nucleic acid can be a gene-specific primer sequence. The gene-specific primer sequences can be designed based on known sequences of a target nucleic acid of interest. The gene-specific primer sequences can span a nucleic acid region of interest, or adjacent (upstream or downstream) of a nucleic acid region of interest.

The length of the gene-specific primer sequence can vary. For example, a gene-specific primer sequence can be, be about, be at least, be at least about, be at most, or be at most about, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values, nucleotides in length. In some aspects, the gene-specific primer sequence is at least 10 nucleotides in length.

The number of the barcode molecules introduced into a partition comprising a gene-specific primer sequence can vary. For example, the number of barcode molecules introduced into a partition comprising a gene-specific primer sequence can be, be about, be at least, be at least about, be at most, or be at most about, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 6000000, 7000000, 8000000, 9000000, 10000000, 20000000, 30000000, 40000000, 50000000, 60000000, 70000000, 80000000, 90000000, 100000000, or a number or a range between any two of these values. In some aspects, the barcode molecules introduced into a partition comprises a set of different gene-specific primer sequences each capable of binding to a specific target nucleic acid sequence.

The number of different gene-specific primer sequences of the barcode molecules introduced into a partition can vary. For example, the number of different gene-specific primer sequences of the barcode molecules introduced into a partition can be, be about, be at least, be at least about, be at most, or be at most about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, 1000, 2000, 3000, 4000, 5000, 6000, 7000, 8000, 9000, 10000, 20000, 50000, 1000000, or a number or a range between any two of these values.

The number of target nucleic acids of interest (e.g. genes of interest) that the barcode molecules introduced into a partition are capable of binding can vary. For example, the number of target nucleic acids of interest (e.g. genes of interest) the barcode molecules introduced into a partition are capable of binding can be, be about, be at least, be at least about, be at most, or be at most about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 200, 300, 400, 500, 600, 700, 800, 900, 1000, 2000, 3000, 4000, 5000, 6000, 7000, 8000, 9000, 10000, 20000, 50000, 1000000, or a number or a range between any two of these values. In some aspects, one barcode molecule introduced into a partition can bind to a molecule (or a copy) of a target nucleic acid. Barcode molecules introduced into a partition can bind to molecules (or copies) of a target nucleic acid or a plurality of target nucleic acids.

In some aspects, the barcode molecules of the plurality of barcode molecules each comprise a poly(dT) sequence, a gene-specific primer sequence, and/or both. The poly(dT) sequence and the gene-specific primer sequence can be on a same barcode molecule or different barcode molecules of the plurality of barcode molecules introduced into a partition.

The ratio of the number of barcode molecules introduced into a partition comprising a poly(dT) sequence and the number of barcode molecules introduced into a partition comprising a gene-specific primer sequence can vary. For example, the ratio can be, be about, be at least, be at least about, be at most, be at most about, 1:100, 1:99, 1:98, 1:97, 1:96, 1:95, 1:94, 1:93, 1:92, 1:91, 1:90, 1:89, 1:88, 1:87, 1:86, 1:85, 1:84, 1:83, 1:82, 1:81, 1:80, 1:79, 1:78, 1:77, 1:76, 1:75, 1:74, 1:73, 1:72, 1:71, 1:70, 1:69, 1:68, 1:67, 1:66, 1:65, 1:64, 1:63, 1:62, 1:61, 1:60, 1:59, 1:58, 1:57, 1:56, 1:55, 1:54, 1:53, 1:52, 1:51, 1:50, 1:49, 1:48, 1:47, 1:46, 1:45, 1:44, 1:43, 1:42, 1:41, 1:40, 1:39, 1:38, 1:37, 1:36, 1:35, 1:34, 1:33, 1:32, 1:31, 1:30, 1:29, 1:28, 1:27, 1:26, 1:25, 1:24, 1:23, 1:22, 1:21, 1:20, 1:19, 1:18, 1:17, 1:16, 1:15, 1:14, 1:13, 1:12, 1:11, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 31:1, 32:1, 33:1, 34:1, 35:1, 36:1, 37:1, 38:1, 39:1, 40:1, 41:1, 42:1, 43:1, 44:1, 45:1, 46:1, 47:1, 48:1, 49:1, 50:1, 51:1, 52:1, 53:1, 54:1, 55:1, 56:1, 57:1, 58:1, 59:1, 60:1, 61:1, 62:1, 63:1, 64:1, 65:1, 66:1, 67:1, 68:1, 69:1, 70:1, 71:1, 72:1, 73:1, 74:1, 75:1, 76:1, 77:1, 78:1, 79:1, 80:1, 81:1, 82:1, 83:1, 84:1, 85:1, 86:1, 87:1, 88:1, 89:1, 90:1, 91:1, 92:1, 93:1, 94:1, 95:1, 96:1, 97:1, 98:1, 99:1, 100:1, or a number or a range between any two of these values.

In some aspectsaspects, a barcode molecule (or each barcode molecule of the plurality of barcode molecules) comprises a template switching oligonucleotide (TSO). A primer comprising a target binding region, such as a poly(dT) sequence, can hybridize to an indexing label and/or a target nucleic acid (e.g., an mRNA) and be extended by, for example, reverse transcription to generate an extended primer comprising a reverse complement of the indexing label and/or the target nucleic acid, or a portion thereof (e.g., cDNA). The extended primer or cDNA can be further extended to include the reverse complement of a TSO oligonucleotide or barcode molecule. The resulting barcoded indexing label or barcoded nucleic acid includes the barcodes of the barcode molecule on the 3'-end.

In some aspects, a barcode molecule does not comprise a TSO. A barcode molecule comprising a target binding region, such as a poly(dT) sequence, can hybridize to an indexing label and/or a target nucleic acid (e.g., an mRNA) and be extended by, for example, reverse transcription to generate an extended primer comprising a reverse complement of the target nucleic acid, or a portion thereof (e.g., cDNA). The extended primer or cDNA can be further extended to include the reverse complement of a template switching oligonucleotide. The resulting barcoded indexing label or barcoded nucleic acid includes the barcodes of the barcode molecule on the 5'-end. The resulting barcoded indexing label or barcoded nucleic acid (e.g., extended cDNA) can comprise a PCR primer binding sequence introduced in the reverse complement of the template switching oligonucleotide.

A TSO is an oligonucleotide that hybridizes to untemplated C nucleotides added by a reverse transcriptase during reverse transcription. The TSO can hybridize to the 3' end of a cDNA molecule. The TSO can include one or more nucleotides with guanine (G) bases on the 3'-end of the TSO, with which the one or more cytosine (C) bases added by a reverse transcriptase to the 3'-end of a cDNA can hybridize. The series of G bases can comprise 1G base, 2 G bases, 3 G bases, 4 G bases, 5 G bases or more than 5 G bases. The series of G bases can be ribonucleotides. The reverse transcriptase can further extend the cDNA using the TSO as the template to generate a barcoded cDNA comprising the TSO. The length of a TSO can vary. For example, a TSO can be, be about, be at least, be at least about, be at most, or be at most about, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values, nucleotides in length.

The number of the barcode molecules introduced into a partition comprising a TSO can vary. In some aspects, the number of barcode molecules introduced into a partition comprising a TSO is, is about, is at least, is at least about, is at most, or is at most about, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, 200000, 300000, 400000, 500000, 600000, 700000, 800000, 900000, 1000000, 2000000, 3000000, 4000000, 5000000, 6000000, 7000000, 8000000, 9000000, 10000000, 20000000, 30000000, 40000000, 50000000, 60000000, 70000000, 80000000, 90000000, 100000000, 200000000, 300000000, 400000000, 500000000, 600000000, 700000000, 800000000, 900000000, 1000000000, or a number or a range between any two of these values.

The TSO of the barcode molecules introduced into a partition can be identical. In some aspects, the TSO of the barcode molecules introduced into a partition is different. The percentage of the barcode molecules of the plurality of barcode molecules introduced into a partition with an identical TSO sequence can be different in different aspects. In some aspects, the percentage of the barcode molecules of the plurality of barcode molecules introduced into a partition with an identical TSO sequence is, is about, is at least, is at least about, is at most, is at most about, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.9%, 100%, or a number or a range between any two of these values.

### Barcoding Indexing Labels and Target Nucleic Acids

The method described herein can comprise barcoding indexing labels and target nucleic acids associated with a cell in the partition (e.g., microwell) using the barcode molecules to generate a barcoded indexing label and a barcoded nucleic acids (e.g., target nucleic acids each hybridized with a barcode molecule, single-stranded barcoded nucleic acids, or double-stranded barcoded nucleic acids).

The method can, in some aspects, further comprises releasing the indexing label and the plurality of target nucleic acids associated with the one or more cells in the partition prior to barcoding the indexing label and the plurality of target nucleic acids. In some aspects, releasing the indexing label and the plurality of target nucleic acids associated with the one or more cells comprises lysing the plurality of cells. For example, prior to barcoding the indexing label and the target nucleic acids, the method can comprise lysing the cells to release the content of the cells within the partition. Lysis agents can be contacted with the cells or cell suspension concurrently. Non-limiting examples of lysis agents include bioactive reagents, such as lysis enzymes, or surfactant based lysis solutions including non-ionic surfactants (e.g., Triton X-100 and Tween 20) and ionic surfactants (e.g., sodium dodecyl sulfate (SDS)). Lysis methods including, but not limited to, thermal, acoustic, electrical, or mechanical cellular disruption can also be used.

### First strand synthesis and single-stranded barcoded nucleic acids

Barcoding the indexing label and the plurality of target nucleic acids can comprise a reverse transcription reaction, for example, to generate a barcoded indexing label and a plurality of barcoded nucleic acids comprising cDNAs. In some aspects, barcoding the indexing label and the plurality of target nucleic acids comprises extending the plurality of barcode molecules using the indexing label and the plurality of target nucleic acids as templates to generate the barcoded indexing label and the plurality of barcoded nucleic acids comprising a plurality of single-stranded barcoded nucleic acids. In some aspects, the plurality of single-stranded barcoded nucleic acids can be hybridized to the plurality of target nucleic acids in the partition.

In some aspects, barcoding target nucleic acids associated with a cell in the partition can comprise extending the barcode molecules using the target nucleic acids as templates to generate partially single-stranded/partially double-stranded barcoded nucleic acids hybridized to the target nucleic acids in the partition (or after target nucleic acids hybridized with barcode molecules are pooled). The partially single-stranded/partially double-stranded barcoded nucleic acids hybridized to target nucleic acids can be separated by denaturation (e.g., heat denaturation or chemical denaturation using for example, sodium hydroxide) to generate single-stranded barcoded nucleic acids of the plurality of barcoded nucleic acids. The single-stranded barcoded nucleic acids can comprise a barcode molecule and an oligonucleotide complementary to the target nucleic acids. In some aspects, the single-stranded barcoded nucleic acids are generated by reverse transcription using a reverse transcriptase. In some aspects, the single-stranded barcoded nucleic acids is generated by using a DNA polymerase.

In some aspects, the method further comprises introducing a plurality of TSO into the partition. Barcoding the plurality of target nucleic acids can comprise extending the plurality of barcode molecules using the plurality of target nucleic acids and the plurality of template switching oligonucleotides as templates to generate the plurality of barcoded nucleic acids comprising a plurality of single-stranded barcoded nucleic acids.

For example, the single-stranded barcoded nucleic acids can be cDNA produced by extending a barcode molecule using a target RNA associated with the cell as a template. The single-stranded barcoded nucleic acids can be further extended using a TSO. The TSO can be introduced into the partitions together with the reverse transcription reagents. For example, a reverse transcriptase can be used to generate a cDNA by extending a barcode molecule hybridized to an RNA. After extending the barcode molecule to the 5'-end of the RNA, the reverse transcriptase can add one or more nucleotides with cytosine (C) bases (e.g. two or three) to the 3'-end of the cDNA. The TSO can include one or more nucleotides with guanine (G) bases (e.g. two or more) on the 3'-end of the TSO. The nucleotides with G bases can be ribonucleotides. The G bases at the 3'-end of the TSO can hybridize to the cytosine bases at the 3'-end of the cDNA. The reverse transcriptase can further extend the cDNA using the TSO as the template to generate a cDNA with the reverse complement of the TSO sequence on its 3'-end. The barcoded nucleic acid can include the barcode sequences (e.g., cell barcode sequence and molecular barcode sequence (e.g., UMI)) on the 5'-end and a TSO sequence at its 3'-end.

In some aspects, barcoding the target nucleic acids comprises extending the barcode molecules using the target nucleic acids as templates and the barcode molecules as TSO to generate single-stranded barcoded nucleic acids that are hybridized to the target nucleic acids. In some aspects, the present method further comprises introducing a plurality of extension primers to the partition. Barcoding the plurality of target nucleic acids can comprise extending the plurality of extension primers using the plurality of target nucleic acids as templates and the plurality of barcode molecules as template switching oligonucleotides to generate the plurality of barcoded nucleic acids comprising a plurality of single-stranded barcoded nucleic acids.

In some aspects , the barcode molecules are not attached to a particle and the barcode molecules can comprise TSO. Extension primers (e.g. oligonucleotides comprising a poly(dT) sequence) can be introduced into the partitions which hybridize to a target nucleic acid (e.g. the poly-adenylated mRNA). The extension primers can be extended using the target nucleic acids as a template. For example, a reverse transcriptase can be used to generate a cDNA by extending an extension primer hybridized to an RNA. After extending the extension primers to the 5'-end of the RNA, the reverse transcriptase can add one or more C bases (e.g. two or three) to the 3'-end of the cDNA. The TSO or barcode molecule can include one or more G bases (e.g. two or more) on the 3'-end of the TSO. The nucleotides with guanine bases can be ribonucleotides. The G bases at the 3'-end of the TSO or barcode molecule can hybridize to the cytosine bases at the 3'-end of the cDNA. The reverse transcriptase can switch template from the mRNA to the TSO or barcode molecule. The reverse transcriptase can further extend the cDNA using the TSO or barcode molecule as the template to generate a cDNA further comprising the reverse complement of the TSO or barcode molecule. In this case, the barcode sequences (e.g., cell barcode sequence and molecular barcode sequence (e.g., UMI)) are on the 3'-end of the generated cDNA.

In some aspects, each of the plurality of single-stranded barcoded nucleic acids is hybridized to one of the plurality of target nucleic acids and one of the plurality of template switching oligonucleotides in the partition.

The single-stranded barcoded nucleic acids can be separated from the template target nucleic acids by digesting the template target nucleic acids (e.g., using RNase), by chemical treatment (e.g., using sodium hydroxide), by hydrolyzing the template target nucleic acids, or via a denaturation or melting process by increasing the temperature, adding organic solvents, or increasing pH. Following the melting process, the target nucleic acids can be removed (e.g. washed away) and the single-stranded barcoded nucleic acids can be retained in the partition (e.g. through attachment to the partitions or through attachments to particles which can be retained in the partitions). In some aspects, the method further comprises removing the plurality of target nucleic acids and the plurality of template switching oligonucleotides hybridized to the single-stranded barcoded nucleic acids. In some aspects, removing the plurality of target nucleic acids comprises denaturation, thermal denaturation, digesting, or hydrolyzing the plurality of target nucleic acids.

In some aspects, each of the plurality of single-stranded barcoded nucleic acid comprises a sequence of a barcode molecule of the plurality of barcode molecules (e.g., an actual sequence of the barcode molecule), a sequence of a target nucleic acid of the plurality of target nucleic acids (e.g. a reverse complement of the target nucleic acid), and/or a sequence of an extension primer of the plurality of extension primers (e.g., an actual sequence of the extension primer).

### Second strand synthesis, amplification, and double-stranded barcoded nucleic acids

The method can further comprise amplifying the barcoded indexing labels and the plurality of barcoded nucleic acids to generate a double-stranded barcoded indexing labels and a plurality of double-stranded barcoded nucleic acids in the partition using the single-stranded barcoded indexing labels and the single-stranded barcoded nucleic acids as templates. The amplifying step can be used to amplify the product of first strand synthesis and/or RT reaction as described here.

For example, barcoding target nucleic acids associated with the cell in the partition (e.g., microwell) can comprise amplifying the barcoded nucleic acids (such as a single-stranded barcoded nucleic acid, or a cDNA generated by using a barcode molecule as disclosed herein). The amplification can comprise generating barcoded nucleic acids comprising double-stranded barcoded nucleic acids in the partition using the single-stranded barcoded nucleic acids as templates. The double-stranded barcoded nucleic acids can be generated from the single-stranded barcoded nucleic acids retained in the partition using, for example, second-strand synthesis or one-cycle PCR. Amplification of the barcoded nucleic acids can include additional cycles of PCR reactions.

The generated double-stranded barcoded nucleic acid can be denaturized or melted to generate two single-stranded barcoded nucleic acids: one single-stranded barcoded nucleic acid retained in the partition (e.g., attached to the particle) and the other single-stranded barcoded nucleic acid released into the solution from the retained single-stranded barcoded nucleic acid that can then be pooled to provide a pooled mixture outside the partitions. Both single-stranded barcoded nucleic acids (e.g. retained in the partitions or pooled outside the partitions) have a sequence comprising a sequence of a barcode molecule (e.g. cell barcode sequence and molecular barcode sequence (e.g., UMI)) and a sequence of a target nucleic acid or a reverse complement thereof.

In some aspects, amplifying the plurality of barcoded nucleic acids comprises amplifying the plurality of barcoded nucleic acids in the partition to generate the plurality of double-stranded barcoded nucleic acids. The plurality of target nucleic acids in a partition can be barcoded and the plurality of barcoded nucleic acids generated are then amplified in the same partition. Further, the plurality of target nucleic acids in a partition can be barcoded and the plurality of barcoded nucleic acids generated are then amplified in the same reaction. For example, the reaction can be a one-step RT-PCR reaction.

Each of the plurality of barcode molecules can comprise a primer sequence. In some aspects, the primer sequence can comprise a PCR primer sequence. Amplifying the plurality of barcoded nucleic acids can comprise amplifying the plurality of barcoded nucleic acids using the primer sequences in single-stranded barcoded nucleic acids of the plurality of single-stranded barcoded nucleic acids, or products thereof.

In some aspects, the barcoding process comprises reverse transcription using an mRNA associated with the cell (and optionally a TSO) as template to generate a barcoded cDNA molecule (optionally with a reverse complement of a TSO) and amplification of the barcoded cDNA by PCR.

### Pooling of Barcoded Indexing Labels and Barcoded Nucleic Acids

The methods disclosed herein can comprise pooling the barcoded indexing labels and the plurality of barcoded nucleic acids, or products thereof, in each of the plurality of partitions to generate pooled barcoded indexing labels and pooled barcoded nucleic acids. Subjecting the plurality of barcoded nucleic acids, or products thereof, to sequencing can comprise subjecting the pooled barcoded nucleic acids, or products thereof, to sequencing. In some aspects, pooling the plurality of barcoded nucleic acids, or products thereof, comprises pooling the plurality of double-stranded barcoded nucleic acids in each of the plurality of partitions to generate the pooled barcoded nucleic acids. For example, the method can comprise pooling the barcoded nucleic acids after barcoding the target nucleic acids and before sequencing the barcoded nucleic acids to obtain pooled barcoded nucleic acids.

In some aspects, pooling barcoded nucleic acids occurs after generating double-stranded barcoded nucleic acids (e.g., after second strand synthesis) or after generating amplified barcoded nucleic acids. The amplified barcoded nucleic acids can be subject to sequencing library construction prior to sequencing. In some aspects, synthesis of single-stranded barcoded nucleic acids and double-stranded barcoded nucleic acids occur after the pooling of target nucleic acids hybridized with barcode molecules.

In some aspects the barcode molecules are attached to particles, only single-stranded barcoded nucleic acids released into bulk (e.g., after amplification of the barcoded nucleic acids) are collected by pooling, and the particles are not pooled (e.g. not removed from the partitions) but retained in the partitions (e.g. by an external magnetic field applied on magnetic beads), thereby allowing one to trace the origin of the pooled barcoded nucleic acids, for example, to its original location in the partitions.

The pooled barcoded nucleic acids can be single-stranded or double-stranded (e.g. generated from the single-stranded pooled barcoded nucleic acids by PCR amplification). The pooled barcoded nucleic acids (e.g. amplified barcoded cDNA) can be purified, and optionally further amplified, prior to sequencing library construction. The pooled barcoded nucleic acids with desired length can be selected.

### Sequencing Library Construction

The barcoded indexing label and the barcoded nucleic acids (e.g. pooled barcoded nucleic acids) can be further processed prior to sequencing to generate processed barcoded indexing label and processed barcoded nucleic acids. For example, the method herein can include amplification of barcoded nucleic acids, fragmentation of amplified barcoded nucleic acids, end repair of fragmented barcoded nucleic acids, A-tailing of fragmented barcoded nucleic acids that have been end-repaired (e.g., to facilitate ligation to adapters), and attaching (e.g., by ligation and/or PCR) with a second sequencing primer sequence (e.g., a Read 2 sequence), sample indexes (e.g. short sequences specific to a given sample library), and/or flow cell binding sequences (e.g., P5 and/or P7). Additional PCR amplification can also be performed. This process can also be referred to as sequencing library construction. In some aspects, separate sequencing libraries are constructed for the barcoded indexing labels and the barcoded nucleic acids.

PCR amplification can be carried out to generate sufficient mass for the subsequent library construction processes. In some aspects, the present method comprises performing a polymerase chain reaction in bulk on the pooled barcoded nucleic acids, or the fragmented barcoded nucleic acids, to generate amplified barcoded nucleic acids. For example, the method can comprise performing a polymerase chain reaction in bulk, subsequent to the pooling, on the pooled barcoded nucleic acids, thereby generating amplified barcoded nucleic acids. In some aspects, performing the polymerase chain reaction in bulk is subsequent to fragmenting the pooled barcoded nucleic acids. The amplification for library preparation can be a separate process from the amplification of the first strand barcoded nucleic acid generated by, for example, the RT reaction as described herein (such as a one-step RT-PCR reaction).

In some aspects, the method comprises fragmenting the pooled barcoded nucleic acids to generate fragmented barcoded nucleic acids to generate fragmented barcoded nucleic acids prior to subjecting the plurality of barcoded nucleic acids, or products thereof, to sequencing. For example, the method can comprise fragmenting (e.g., via enzymatic fragmentation, mechanical force, chemical treatment, etc.) the pooled barcoded nucleic acids to generate fragmented barcoded nucleic acids. Fragmentation can be carried out by any suitable process such as physical fragmentation, enzymatic fragmentation, or a combination of both. For example, the barcoded nucleic acids can be sheared physically using acoustics, nebulization, centrifugal force, needles, or hydrodynamics. The barcoded nucleic acids can also be fragmented using enzymes, such as restriction enzymes and endonucleases.

Fragmentation yields fragments of a desired size for subsequent sequencing. The desired sizes of the fragmented nucleic acids are determined by the limitations of the next generation sequencing instrumentation and by the specific sequencing application as will be understood by a person skilled in the art. For example, when using Illumina technology, the fragmented nucleic acids can have a length of between about 50 bases to about 1,500 bases. In some aspects, the fragmented barcoded nucleic acids have about 100 bp to 700 bp in length.

Fragmented barcoded nucleic acids can undergo end-repair and A-tailing (to add one or more adenine bases) to form an A overhang. This A overhang allows adapter containing one or more thymine overhanging bases to base pair with the fragmented barcoded nucleic acids.

Fragmented barcoded nucleic acids can be further processed by adding additional sequences (e.g. adapters) for use in sequencing based on specific sequencing platforms. Adapters can be attached to the fragmented barcoded nucleic acids by ligation using a ligase and/or PCR. For example, fragmented barcoded nucleic acids can be processed by adding a second sequencing primer sequence. The second sequencing primer sequence can comprise a Read 2 sequence. An adapter comprising the second primer sequence can be ligated to the fragmented barcoded nucleic acids after, for example, end-repair and A tailing, using a ligase. The adaptor can include one or more thymine (T) bases that can hybridize to the one or more A bases added by A tailing. An adaptor can be, for example, partially double-stranded or double stranded. In some aspects, the amplified barcoded nucleic acids comprise a sequencing primer sequence.

The adapter can also include platform-specific sequences for fragment recognition by specific sequencing instrument. In some aspects, the amplified barcoded nucleic acids comprise a sequence for attaching the amplified barcoded nucleic acids to a flow well. For example, the amplified barcoded nucleic acids can comprise an adapter that comprises a sequence for attaching the fragmented barcoded nucleic acids to a flow well of Illumina platforms, such as a P5 sequence, a P7 sequence, or a portion thereof. Different adapter sequences can be used for different next generation sequencing instrument as will be understood by a person skilled in the art.

The adapter can also contain sample indexes to identify samples and to permit multiplexing. Sample indexes enable multiple samples to be sequenced together (i.e. multiplexed) on the same instrument flow cell as will be understood by a person skilled in the art. Adapters can comprise a single sample index or a dual sample indexes depending on the implementations such as the number of libraries combined and the level of accuracy desired.

In some aspects, the amplified barcoded nucleic acids generated from sequencing library construction can include a P5 sequence, a sample index, a Read 1 sequence, a cell barcode sequence, a molecular barcode sequence (e.g., UMI), a poly(dT) sequence, a target biding region, a sequence of a target nucleic acid or a portion thereof, a Read 2 sequence, a sample index, and/or a P7 sequence (e.g., from 5'-end to 3'-end). In some aspects, the amplified barcoded nucleic acids can include a P5 sequence, a sample index, a Read 1 sequence, a cell barcode sequence, a molecular barcode sequence (e.g., UMI), a sequence of a template switching oligonucleotide, a sequence of a target nucleic acid or a portion thereof, a Read 2 sequence, a sample index, and/or a P7 sequence (e.g., from 5'-end to 3'-end).

Sequencing the barcoded nucleic acids, or products thereof, can comprise sequencing products of the barcoded nucleic acids. Products of the barcoded nucleic acids can include the processed nucleic acids generated by any step of the sequencing library construction process, such as amplified barcoded nucleic acids, fragmented barcoded nucleic acids, fragmented barcoded nucleic acids comprising additional sequences such as the second sequencing primer sequence and/or adapter sequences described herein.

### Sequencing Barcoded Indexing Labels and Barcoded Nucleic Acids

The method disclosed herein can comprise sequencing the barcoded indexing labels and the barcoded nucleic acids or products thereof to obtain nucleic acid sequences of the barcoded indexing label and the barcoded nucleic acids. The barcoded nucleic acids generated by the method disclosed herein can comprise barcoded nucleic acids pooled, from each partition, into a pooled mixture outside the partitions. The barcoded nucleic acids retained in a partition and the pooled barcoded nucleic acids in a pooled mixture outside the partitions can be sequenced using a same or different sequencing techniques.

In some aspects, sequencing the plurality of barcoded nucleic acids (or the barcoded indexing labels) or products thereof comprises sequencing the pooled barcoded nucleic acids (or the pooled barcoded indexing labels) to obtain nucleic acid sequences of the pooled barcoded nucleic acids (or the pooled barcoded indexing labels). As used herein, a "sequence" can refer to the sequence, a complementary sequence thereof (e.g., a reverse, a compliment, or a reverse complement), the full-length sequence, a subsequence, or a combination thereof. The nucleic acids sequences of the pooled barcoded nucleic acids (or the pooled barcoded indexing labels) can each comprise a sequence of a barcode molecule (e.g., the cell barcode sequence and the molecular barcode sequence (e.g., UMI)) and a sequence of a target nucleic acid (or an indexing label) associated with the cell or a reverse complement thereof.

Pooled barcoded nucleic acids (or pooled barcoded indexing labels) can be sequenced using any suitable sequencing method identifiable. For example, sequencing the pooled barcoded nucleic acids can be performed using high-throughput sequencing, pyrosequencing, sequencing-by-synthesis, single-molecule sequencing, nanopore sequencing, sequencing-by-ligation, sequencing-by-hybridization, next generation sequencing, massively-parallel sequencing, primer walking, and any other sequencing methods known in the art and suitable for sequencing the barcoded nucleic acids generated using the methods herein described.

### Analysis

Method disclosed herein can comprise determining a profile of the cells simultaneously from multiple samples, for example from the sequence of the barcode nucleic acids. The obtained nucleic acid sequences of the plurality of barcoded nucleic acids (e.g. nucleic acid sequences of pooled barcoded nucleic acids) can be subjected to any downstream post-sequencing data analysis as will be understood by a person of skill in the art. The sequence data can undergo a quality control process to remove adapter sequences, low-quality reads, uncalled bases, and/or to filter out contaminants. The high-quality data obtained from the quality control can be mapped or aligned to a reference genome or assembled de novo.

Profile analysis, for example gene expression quantification and differential expression analysis, can be carried out to identify genes whose expression differs in different cells. Barcoded nucleic acids from a cell can have an identical cell barcode sequence in the sequencing data and can be identified. Barcoded nucleic acids from different cells can have different cell barcode sequences in the sequencing data and can be identified. Barcoded nucleic acids with an identical cell barcode sequence, an identical target sequence, and different molecular barcode sequences in the sequencing data can be quantified and used to determine the expression of the target.

The method can, for example, comprise determining a profile (e.g. an expression profile, a transcription profile, an omics profile, or a multi-omics profile) of the one or more cells from the sequences of the barcoded nucleic acids. In some aspects, the profile comprises a single omics profile, such as a transcriptome profile. In some aspects, the profile comprises a multi-omics profile, which can include profiles of genome (e.g. a genomics profile), pro-teome (e.g. a proteomics profile), transcriptome (e.g. a transcriptomics profile), epigenome (e.g. an epigenomics profile), metabolome (e.g. a metabolomics profile), and/or microbiome (e.g. microbiome profile). In some aspects, the multi-omics profile comprises a genomics profile, a proteomics profile, a transcriptomics profile, an epigenomics profile, a metabolomics profile, a chromatics profile, a protein expression profile, a cytokine secretion profile, or a combination thereof.

The profile can comprise an expression of a target nucleic acid of the plurality of target nucleic acids. For example, the expression of the target nucleic acid can comprise an abundance of the target nucleic acid. The abundance of the target nucleic acid can comprise an abundance of molecules of the target nucleic acid barcoded using the barcode molecules. The abundance of the molecules of the target nucleic acid can comprise a number of occurrences of the molecules of the target nucleic acid. In some aspects, the number of occurrences of the molecules of the target nucleic acid is, is indicated by, or is determined using, a number of the barcoded nucleic acids comprising a sequence of the target nucleic acid and different molecular barcode sequences in the sequences of the barcoded nucleic acids. In some aspects, the profile includes an RNA expression profile and/or a protein expression profile. The expression profile can comprise an RNA expression profile, an mRNA expression profile, and/or a protein expression profile. A profile can also be a profile of one or more target nucleic acids (e.g. gene markers) or a selection of genes associated with the cell. In some aspects, target nucleic acids associate with a cell (e.g., a living cell) can be analyzed in a high-throughput manner by the present method.

### Cells

The cells can be obtained from any organism of interest. A cell can be, for example, a mammalian cell, and particularly a human cell such as T cells, B cells, natural killer cells, stem cells, or cancer cells.

Cells described herein can be obtained from, derived from, cultured from, or progenies of cells cultured from a cell sample. A cell sample comprising cells can be obtained from any source including a clinical sample and a derivative thereof, a biological sample and a derivative thereof, a forensic sample and a derivative thereof, and a combination thereof. A cell sample can be collected from any bodily fluids including, but not limited to, blood, urine, serum, lymph, saliva, anal, and vaginal secretions, perspiration and semen of any organism. A cell sample can be products of experimental manipulation including purification, cell culturation, cell isolation, cell separation, cell quantification, sample dilution, or any other cell sample processing approaches. A cell sample can be obtained by dissociation of any biopsy tissues of any organism including, but not limited to, skin, bone, hair, brain, liver, heart, kidney, spleen, pancreas, stomach, intestine, bladder, lung, esophagus.

In some aspects, the cell sample is a clinical sample or a derivative thereof, a biological sample or a derivative thereof, an environmental sample or a derivative thereof, a forensic sample or a derivative thereof, or a combination thereof. In some aspects, the cell sample is collected from blood, urine, serum, lymph, saliva, anal, and vaginal secretions, perspiration, and/or semen of any organism. In some aspects, the cell sample is obtained from skin, bone, hair, brain, liver, heart, kidney, spleen, pancreas, stomach, intestine, bladder, lung, and/or esophagus of any organism.

In some aspects, the cells are cultured cells, such as cells from a cultured cell line. In some aspects, the cells comprise immune cells, fibroblast cells, stem cells, or cancer cells. In some aspects, the cells are obtained from, cultured from, or progenies of cells cultured from a cell sample of a disease or disorder disclosed herein.

The cells can be cancer cells. Examples of cancer cells include, but are not limited to, bladder cancer cells (e.g., CRL-1472, CRL-1473, CRL-1749, CRL-2169, HTB-2, HTB-4, HTB-5, HTB-9), breast cancer cells (e.g., MCF-7, CRL-1897, CRL-1902, CRL-2983, CRL-2988, CRL-3127, CRL-3166, CRL-1897, CRL-3180), colon cancer cells (e.g., CCL-229, CCL-233, CCL-235, CCL-237, CCL-248, CCL-255, CRL-5792, HTB-37, HTB-39), endometrial cancer cells (e.g., CRL-1671), gastric cancer cells (e.g., CRL-1739, CRL-5822, CRL-5971, CRL-5973, CRL-5974, HTB-103, MKN-28, SNU638), leukemia cells (e.g., NB4, CCL-119, CCL-240, CCL-243, CRL-1582, CRL-1873, CRL-2724, TIB-202), liver cancer cells (e.g., CRL-2234, CRL-2236, CRL-2237, CRL-2238, CRL-8024, CRL-10741, HTB-52, HB-8065), lung cancer cells (e.g., CCL-256, CCL-257, CRL-5803, CRL-5872, CRL-5875, CRL-5877, CRL-5908, HTB-183), lymphoma cells (e.g., U937), small cell lung cancer cells (CRL-11350), non-small cell lung cancer cells (e.g., A549, CRL-5803, CRL-5893, CRL-5908, CRL-9609, HTB-178), kidney cancer cells (e.g., CRL-7569, CRL-7629, HTB-46, HTB-47) ovarian (e.g., SKOV3, CRL-1572, HTB-75, HTB-78), pancreatic cancer cells (e.g., CRL-1682, CRL-1687, CRL-1918, CRL-1997, CRL-2172, CRL-2547, HTB-79, HTB-80), prostate cancer cells (e.g., CRL-1740, CRL-3031, CRL-3033, CRL-3314, CRL-3315, CRL-3470, HTB-81), and skin cancer cells (e.g., A-375, HTB-66, HTB-69, HTB-71, CRL-7724).

The cells can be, for example, cells suitable for studying a cardiovascular disease (e.g., CRL-1395, CRL-1444, CRL-1476, CRL-1730, CRL-1999, CRL-2018, and CRL-2581), diabetes (e.g., CRL-3237, CRL-3242, CRL-11506, PCS-210-010), an infectious disease (e.g. CCL-86, CCL-156, CCL-214), a neurodegenerative disease (e.g., ACS-5001, ACS-1013, CRL-2541, HTB-11), or a respiratory disease (e.g., PCS-301-011, PCS-301-013, CRL-1848, CRL-4051, CRL-9609). In some aspects, the cells comprise A549 cells, NB4 cells, U937 cells, or a combination thereof. In some aspects, the cells comprise living cells.

### Kit

Disclosed herein include a kit for indexing a plurality of samples. The kit can, in some aspects, comprise a coupling agent comprising a coupling group and a first reactive group; for each of the plurality of samples, a plurality of indexing labels each comprising an identical sample-specific-indexing sequence and a second reactive group capable of forming a covalent bond with the first reactive group of the coupling agent; and instructions to use the kit for indexing multiple samples according to any one of the methods disclosed herein for indexing multiple samples. Also disclosed herein include a kit for analyzing nucleic acids in a plurality of samples. The kit can, in some aspects, comprises a coupling agent each comprising a coupling group and a first reactive group; for each of the plurality of samples, a plurality of indexing labels each comprising an identical sample-specific-indexing sequence and a second reactive group capable of forming a covalent bond with the first reactive group of the coupling agent; a plurality of beads, wherein each bead is attached to, reversibly attached to, covalently attached to, or irreversibly attached to a plurality of barcode molecules, and wherein each barcode molecule of the plurality of barcode molecules comprises a cell barcode sequence, a molecular label sequence, a primer sequence, a primer binding site, a template switching oligonucleotide, or a combination thereof; and instructions to use the kit for indexing multiple samples according to any one of the methods disclosed herein for indexing multiple samples.

The kit can, in some aspects, further comprise a plurality of partitions comprising, for example, at least 100 partitions (e.g., droplets or wells). The kit can, in some aspects, comprise one or more reagents for use in the method disclosed herein. For example, the kit can further comprise one or more of cell lysis agents, enzymes (such as reverse transcriptase, polymerase), and chemical reagents.

### EXAMPLES

Some aspects discussed above are disclosed in further detail in the following example, which are not in any way intended to limit the scope of the present disclosure.

### Example 1

### Single-Cell RNA Sequencing

For multiplexed single cell RNA-seq, modified sample-specific oligos were used as sample barcodes to label cell from different samples.

### Label preparation

5'NHS-PEG5-Tz modified sample-specific oligos were used to tag multiple samples before sample pooling for multiplexed scRNA-seq. The sample-specific oligo sequence consists of a PCR handle sequence, a well position specific barcode, a random DNA sequence as unique molecular index (UMI) and an poly(A) primer sequence.

A sample index oligonucleotide is a single-stranded DNA (ssDNA) having a sequence as follows:

TGTCAAGATGCTACCGTTCAGAGTGACCTGCTTCACGCAAAAAAAAAAAAAAAAAAAA
A (SEQ ID NO:1).

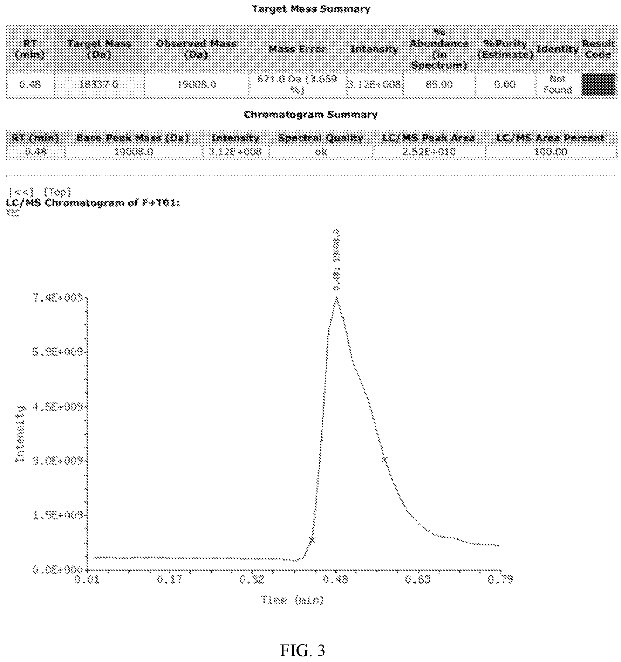

NH2-C6-ssDNA was incubated with NHS-PEG5-Tz to prepare ssDNA-Tz. The efficiency of small molecule modification during label preparation was evaluated using mass spectrometry (FIG. 3).

### Cell labeling

Cell labeling was carried out as follows:
1. AG49, NB4, U937 cell lines were washed with PBS, resuspended in PBS, and centrifuged at 400xg. The supernatant was removed.
2. Pre-labeling of cells. NHS-PEG4-TCO was used as a pre-labeling reagent to label and resuspend the washed cells. The pre-labeling reaction was carried out for 10 minutes in the dark at room temperature. An equal volume of FBS was then added to quench the reaction for 10 minutes.
3. Washing with PBS. After quenching, the cells were centrifuged for 2 min at 400xg to discard the supernatant, and then washed twice with PBS, the same as step 2.
4. Sample specific oligonucleotide tag. 20 µL of the specific ssDNA-Tz reagent was added to the cell pellet and resuspend. The labeling reaction was carried out for 30 minutes in the dark at room temperature, and the cells were resuspended every 15 minutes. During this period, quenching reagent was prepared with PBS 5 µL of quenching reagent was added to the cells after labeling, and reacted for 10 minutes at room temperature in the dark.
6. Washing the cells. After quenching, the cells were centrifuged for 2 minutes at 400xg to discard the supernatant, and were then washed twice with PBS, the same as 2.
7. Counting and pooling the cells from three cell line. A cell counter was used to detect the concentration and viability of the cells. The three cell lines were pooled in a 1:1:1 ratio, and were then analyzed with single-cell sequencing process.

### Single-cell sequencing

GEXSCOPE^{®} Single Cell RNAseq Library Construction kit (Singleron Biotechnologies) was used to demonstrate the technical feasibility and the utility of the present disclosure in high-throughput multiplexed single-cell RNA sequencing. The experiment was conducted according to manufacturer's instructions with modifications described below.

Briefly, cells were labeled as described in the above steps. After sample pooling, cell suspensions were loaded onto the microchip to partition single cells into individual wells on the chip. Cell barcoding magnetic beads were then loaded to the microchip and washed. Each cell-barcoding magnetic bead contains oligos with a unique cell barcode sequence combined with oligo-dT on the surface. Each oligo on the bead also has a unique molecule index sequence (UMI); the number of UMIs detected in the sequence can be used to accurately quantify different RNA molecules. Only one bead can fall into each well on the microchip based on the diameters of the beads and well (about 30 µm and 40 µm, respectively). Then 100 µL reaction mixture was loaded into the chip, and the chip was incubated on ice for 10 minutes to lyse the cells. After the cells are lysed, the magnetic beads, together with captured RNAs, were taken out of the microchip and subject to reverse transcription.

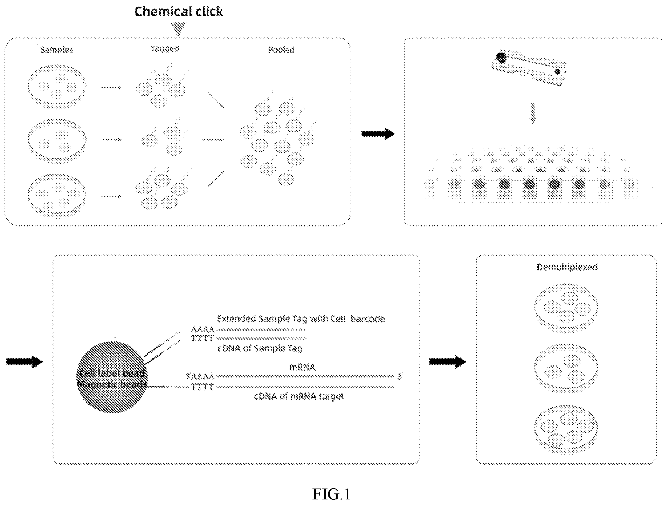

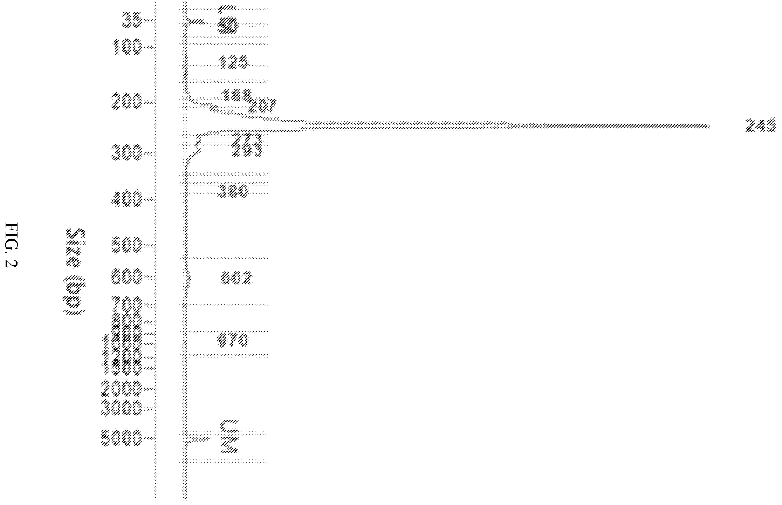

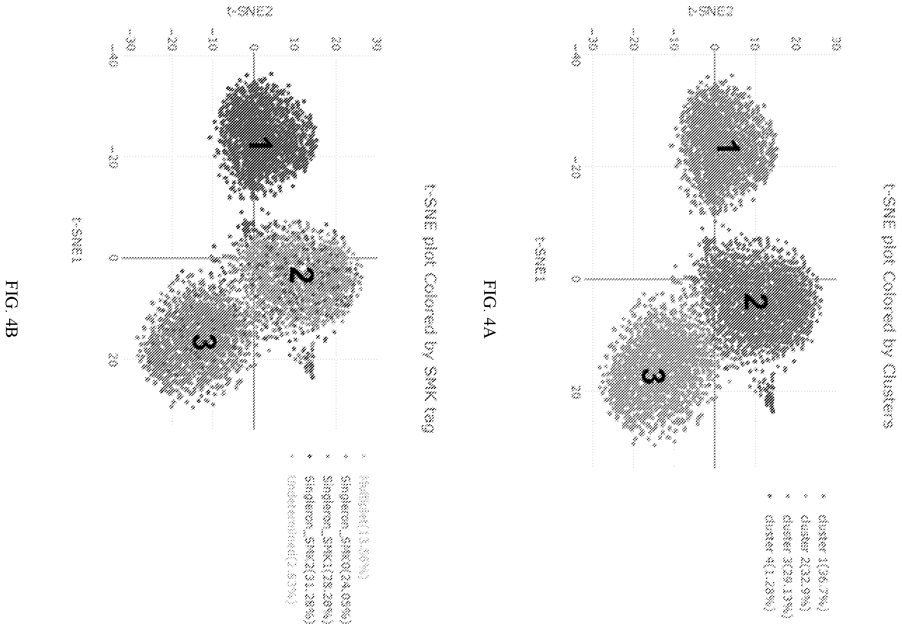

After the reverse transcription is completed, sample labels were recovered through heat shock reaction. The magnetic beads were washed with wash buffer, and were then resuspend in TE buffer. The beads were placed at 95°C for 5 minutes, and were then quickly placed on the magnetic stand to recover the supernatant. The supernatant was used as a template for PCR amplification to construct tag library. The remaining magnetic beads were also used as a template for PCR amplification. The cDNA was then used to construct a transcriptome sequencing library. The resulting RNA-seq library was sequenced on an Illumina Nova-Seq with PE150 mode and analyzed with CeleScope bioinformatics workflow (Singleron Biotechnologies), as shown in FIG. 1. The Tag amplified library was assessed by quality control peak map (FIG. 2). The efficiency of label splitting was evaluated through combined analysis of T-distributed stochastic neighbor embedding (t-SNE) plot colored by cluster and by Tag (FIGS. 4A-4B) .

### Terminology

In at least some of the previously described aspects, one or more elements used in an aspect can interchangeably be used in another aspect unless such a replacement is not technically feasible. It will be appreciated by those skilled in the art that various other omissions, additions and modifications may be made to the methods and structures described above.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (*e.g*., bodies of the appended claims) are generally intended as "open" terms (*e.g.,* the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to aspects containing only one such recitation, even when the same claim includes the introductory phrases "one or more" or "at least one" and indefinite articles such as "a" or "an" (*e.g.,* "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g*., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g*., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g*., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

## Claims

1. A method of indexing a plurality of samples, comprising:
(a) providing a plurality of samples, wherein each of the plurality of samples comprises a plurality of cells, wherein the plurality of cells comprises living cells and is derived from cell separation;
(b) for each of the plurality of samples, contacting a coupling agent with the sample, wherein the coupling agent comprises a coupling group and a first reactive group, thereby associating the coupling agent to the surface of the plurality of cells; and
(c) for each of the plurality of samples, contacting a plurality of indexing labels each comprising an identical sample-specific-indexing sequence and a second reactive group capable of forming a covalent bond with the first reactive group of the coupling agent in an inverse electron demand Diels-Alder (IEDDA) reaction, thereby generating a plurality of cells each associated with the sample-specific-indexing label, wherein the sample-specific-indexing sequence for each sample is different from other samples.

2. A method of analyzing nucleic acids, comprising:
(a) providing a plurality of samples, wherein each of the plurality of samples comprises a plurality of cells, wherein the plurality of cells comprises living cells and is derived from cell separation;
(b) for each of the plurality of samples, contacting a coupling agent with the sample, wherein the coupling agent comprises a coupling group and a first reactive group, thereby associating the coupling agent to the surface of the plurality of cells;
(c) for each of the plurality of samples, contacting a plurality of indexing labels each comprising an identical sample-specific-indexing sequence and a second reactive group capable of forming a covalent bond with the first reactive group of the coupling agent in an inverse electron demand Diels-Alder (IEDDA) reaction, thereby generating a plurality of cells each associated with the sample-specific-indexing label, wherein the sample-specific-indexing sequence for each sample is different from other samples;
(d) pooling the plurality of cells associated with the sample-specific-indexing labels from the plurality of samples to form a pooled sample with a plurality of pooled cells;
(e) partitioning the plurality of pooled cells into a plurality of partitions, thereby at least 25% of the plurality of partitions each comprises a single cell of the plurality of pooled cells;
(f) analyzing target nucleic acids associated with the single cell, wherein the target nucleic acids comprise the indexing labels associated with the single cell, and
(g) determining the sample origin of single cell based on the sample-specific-indexing sequence associated with the single cell.

3. The method of claim 2, wherein at least 75% of the plurality of partitions each comprises a single cell of the plurality of pooled cells, and/or wherein the plurality of partitions comprise droplets or microwells.

4. The method of any one of claims 2-3, wherein determining the sample origin of single cell comprises high temperature denaturation, fragment sorting, or a combination thereof.

5. The method of any one of claims 2-4, wherein the target nucleic acids comprise:
(1) cellular nucleic acids, viral nucleic acids, bacterial nucleic acids, mitochondrial nucleic acids, synthetic nucleic acids, or amplification product thereof, or a combination thereof; and/or
(2) deoxyribonucleic acid (DNA), ribonucleic acid (RNA), or a combination thereof, and/or
(3) poly-adenylated messenger ribonucleic acid (mRNAs) of the single cell.

6. The method of any one of claims 2-5, wherein analyzing target nucleic acids comprises barcoding in the plurality of partitions comprising a single cell, using a plurality of barcode molecules in a single partition, to generate barcoded target nucleic acids, optionally wherein:
(1) barcoding the target nucleic acids comprises:
(a) barcoding (i) the indexing labels associated with the single cell and (ii) mRNAs of the single cells to generate (i-a) a barcoded indexing label and (ii-a) barcoded cDNAs, and/or
(b) a reverse transcription reaction, and the barcoded targeted nucleic acids comprises complementary deoxyribonucleic acid (cDNA);
(2) a barcode molecule of the plurality of barcode molecules comprises a cell barcode sequence, a molecular label sequence, a primer sequence, a primer binding site, a template switching oligonucleotide, or a combination thereof, further optionally wherein:
(a) the molecular label sequences comprise unique molecule identifiers (UMls),
(b) the molecular label sequence is 2-40 nucleotides in length, and/or
(c) the primer sequence is a sequencing primer sequence, further optionally wherein the sequencing primer sequence is a Read 1 sequence, a Read 2 sequence, or a portion thereof;
(3) the barcode molecules of the plurality of barcode molecules in a single partition comprise an identical cell barcode sequence and different molecular label sequence; and/or
(4) in each of the plurality of partitions comprising a single cell, the plurality of barcode molecules are attached to, reversibly attached to, covalently attached to, or irreversibly attached to a bead, further optionally wherein:
(a) barcoding in a single partition comprises partitioning the bead into the single partition,
(b) partitioning the plurality of pooled cells into a plurality of partitions comprises co-partitioning the pooled cells and the bead into the single partitions,
(c) the bead is a solid bead, and/or
(d) the bead is a magnetic bead or a polymer bead.

7. The method of any one of claims 2-6, wherein analyzing target nucleic acids comprises:
(1) sequencing the target nucleic acids, amplification products thereof, or a portion of the target nucleic acids or amplification products thereof;
(2) introducing a plurality of template switching oligonucleotides into the partition and barcoding the plurality of target nucleic acids by extending the plurality of barcode molecules using the target nucleic acids and the plurality of template switching oligonucleotides as templates to generate barcoded nucleic acids; and/or
(3) introducing a plurality of extension primers to the partition and barcoding the target nucleic acids by extending the plurality of extension primers using the target nucleic acids as templates and the plurality of barcode molecules as template switching oligonucleotides to generate barcoded nucleic acids.

8. The method of any one of claims 1-7,
(1) wherein the coupling group of the coupling agent is capable of forming a covalent bond with the surface of the plurality of cells,
(2) wherein the coupling agent and/or the indexing label further comprises a hydrophilic group,
(3) wherein the hydrophilic groups comprise PEG,
(4) wherein the coupling agent is NHS-PEG4-TCO, and/or
(5) wherein the coupling group of the coupling agent is capable of forming a covalent bond with -NH₂ on the surface of the plurality of cells.

9. The method of any one of claims 1-8, wherein at least two cells from a same sample are labeled with identical sample-specific-indexing sequences.

10. The method of any one of claims 1-9,
(1) wherein each of the indexing labels is a 5' NHS-PEG5-Tz modified oligonucleotide,
(2) wherein each of the indexing labels further comprises a PCR handle sequence, a UMI, a capture sequence, or a combination thereof, optionally wherein the capture sequence comprises a poly(dA) sequence, and/or
(3) wherein the indexing labels are single-stranded DNA.

11. The method of any one of claims 1-10, wherein one of the first reactive group and the second reactive group comprises a tetrazine (Tz) group and the other comprises a trans-cyclooctene (TCO) group.

12. The method of any one of claim 1-11,
(1) wherein the plurality of samples comprises at least 12 samples, optionally wherein the samples are clinical samples, environmental samples, biological samples, or a combination thereof; and/or
(2) wherein the plurality of cells comprises prokaryotic cells, eukaryotic cells, or a combination thereof.

13. The method of any one of claims 1-12, comprising washing
(1) the plurality of cells before step (a),
(2) the plurality of cells to remove unbound coupling agent after step (b) and before step (c), and/or
(3) the cells associated with the sample-specific-indexing labels to remove unbound sample-specific-indexing labels after step (c); and/or resuspending the cells in aqueous solution.

## Patentansprüche

1. Verfahren zur Indizierung einer Vielzahl von Proben, das Folgendes aufweist:
(a) Bereitstellen einer Vielzahl von Proben, wobei jede der Vielzahl von Proben eine Vielzahl von Zellen aufweist, wobei die Vielzahl von Zellen lebende Zellen beinhaltet und aus einer Zellseparation stammt;
(b) für jede der Vielzahl von Proben Inkontaktbringen eines Kopplungsreagenzes mit der Probe, wobei das Kopplungsreagenz eine Kopplungsgruppe und eine erste reaktive Gruppe aufweist, wodurch das Kopplungsreagenz mit der Oberfläche der Vielzahl von Zellen assoziiert wird; und
(c) für jede der Vielzahl von Proben Inkontaktbringen einer Vielzahl von Indexierungslabels, die jeweils eine identische probenspezifische Indexierungssequenz und eine zweite reaktive Gruppe aufweisen, die in der Lage ist, in einer Inverse-Elektronenbedarfs-Diels-Alder-(IEDDA)-Reaktion eine kovalente Bindung mit der ersten reaktiven Gruppe des Kopplungsreagenzes zu bilden, wodurch eine Vielzahl von Zellen erzeugt wird, die jeweils mit dem probenspezifischen Indexierungslabel assoziiert sind, wobei sich die probenspezifische Indexierungssequenz für jede Probe von denjenigen anderer Proben unterscheidet.

2. Verfahren zur Analyse von Nukleinsäuren, das Folgendes aufweist:
(a) Bereitstellen einer Vielzahl von Proben, wobei jede der Vielzahl von Proben eine Vielzahl von Zellen aufweist, wobei die Vielzahl von Zellen lebende Zellen beinhaltet und aus einer Zellseparation stammt;
(b) für jede der Vielzahl von Proben Inkontaktbringen eines Kopplungsreagenzes mit der Probe, wobei das Kopplungsreagenz eine Kopplungsgruppe und eine erste reaktive Gruppe aufweist, wodurch das Kopplungsreagenz mit der Oberfläche der Vielzahl von Zellen assoziiert wird;
(c) für jede der Vielzahl von Proben Inkontaktbringen einer Vielzahl von Indexierungslabels, die jeweils eine identische probenspezifische Indexierungssequenz und eine zweite reaktive Gruppe aufweisen, die in der Lage ist, in einer Inverse-Elektronenbedarfs-Diels-Alder-(IEDDA)-Reaktion eine kovalente Bindung mit der ersten reaktiven Gruppe des Kopplungsreagenzes zu bilden, wodurch eine Vielzahl von Zellen erzeugt wird, die jeweils mit dem probenspezifischen Indexierungslabel assoziiert sind, wobei sich die probenspezifische Indexierungssequenz für jede Probe von denjenigen anderer Proben unterscheidet;
(d) Poolen der Vielzahl von Zellen, die mit den probenspezifischen Indexierungslabels assoziiert sind, aus der Vielzahl von Proben, um eine gepoolte Probe mit einer Vielzahl gepoolter Zellen zu bilden;
(e) Partitionieren der Vielzahl gepoolter Zellen in eine Vielzahl von Partitionen, wobei mindestens 25 % der Vielzahl von Partitionen jeweils eine einzelne Zelle der Vielzahl gepoolter Zellen aufweisen;
(f) Analysieren von Zielnukleinsäuren, die mit der einzelnen Zelle assoziiert sind, wobei die Zielnukleinsäuren die mit der einzelnen Zelle assoziierten Indexierungslabels beinhalten; und
(g) Bestimmen der Probenherkunft der einzelnen Zelle auf der Grundlage der mit der einzelnen Zelle assoziierten probenspezifischen Indexierungssequenz.

3. Verfahren nach Anspruch 2, wobei zumindest 75 % der Vielzahl von Partitionen jeweils eine einzelne Zelle der Vielzahl gepoolter Zellen aufweisen, und/oder wobei die Vielzahl von Partitionen Tröpfchen oder Mikrokavitäten aufweist.

4. Verfahren nach einem der Ansprüche 2-3, wobei das Bestimmen der Probenherkunft der einzelnen Zelle eine Hochtemperaturdenaturierung, Fragmentsortierung oder eine Kombination davon beinhaltet.

5. Verfahren nach einem der Ansprüche 2-4, wobei die Zielnukleinsäuren Folgendes beinhalten:
(1) zelluläre Nukleinsäuren, virale Nukleinsäuren, bakterielle Nukleinsäuren, mitochondriale Nukleinsäuren, synthetische Nukleinsäuren oder Amplifikationsprodukte davon oder eine Kombination davon; und/oder
(2) Desoxyribonukleinsäure (DNA), Ribonukleinsäure (RNA) oder eine Kombination davon; und/oder
(3) polyadenylierte Messenger-Ribonukleinsäuren (mRNAs) der einzelnen Zelle.

6. Verfahren nach einem der Ansprüche 2-5, wobei das Analysieren der Zielnukleinsäuren ein Barcodieren in der Vielzahl von Partitionen, die eine einzelne Zelle aufweisen, unter Verwendung einer Vielzahl von Barcode-Molekülen in einer einzelnen Partition beinhaltet, um barcodierte Zielnukleinsäuren zu erzeugen, optional wobei:
(1) das Barcodieren der Zielnukleinsäuren Folgendes beinhaltet:
(a) Barcodieren (i) der mit der einzelnen Zelle assoziierten Indexierungslabels und (ii) der mRNAs der einzelnen Zelle, um (i-a) ein barcodiertes Indexierungslabel und (ii-a) barcodierte cDNAs zu erzeugen; und/oder
(b) eine Reverse-Transkriptionsreaktion, und wobei die barcodierten Zielnukleinsäuren komplementäre Desoxyribonukleinsäure (cDNA) beinhalten;
(2) ein Barcode-Molekül der Vielzahl von Barcode-Molekülen eine Zell-Barcode-Sequenz, eine molekulare Markierungssequenz, eine Primersequenz, eine Primerbindungsstelle, ein Template-Switching-Oligonukleotid oder eine Kombination davon aufweist, ferner optional wobei:
(a) die molekularen Markierungssequenzen Unique Molecular Identifiers (UMIs) beinhalten,
(b) die molekulare Markierungssequenz eine Länge von 2-40 Nukleotiden aufweist; und/oder
(c) die Primersequenz eine Sequenzierungsprimersequenz ist, ferner optional wobei die Sequenzierungsprimersequenz eine Read-1-Sequenz, eine Read-2-Sequenz oder einen Teil davon darstellt;
(3) die Barcode-Moleküle der Vielzahl von Barcode-Molekülen in einer einzelnen Partition eine identische Zell-Barcode-Sequenz und unterschiedliche molekulare Markierungssequenzen aufweisen; und/oder
(4) in jeder der Vielzahl von Partitionen, die eine einzelne Zelle aufweisen, die Vielzahl von Barcode-Molekülen an ein Bead gebunden, reversibel an ein Bead gebunden, kovalent an ein Bead gebunden oder irreversibel an ein Bead gebunden ist, ferner optional wobei:
(a) das Barcodieren in einer einzelnen Partition das Einbringen des Beads in die einzelne Partition beinhaltet,
(b) das Partitionieren der Vielzahl gepoolter Zellen in eine Vielzahl von Partitionen das gemeinsame Partitionieren der gepoolten Zellen und des Beads in die einzelnen Partitionen beinhaltet,
(c) das Bead ein festes Bead ist; und/oder
(d) das Bead ein magnetisches Bead oder ein Polymerbead ist.

7. Verfahren nach einem der Ansprüche 2-6, wobei das Analysieren der Zielnukleinsäuren Folgendes beinhaltet:
(1) Sequenzieren der Zielnukleinsäuren, von Amplifikationsprodukten davon oder eines Teils der Zielnukleinsäuren oder Amplifikationsprodukte davon;
(2) Einbringen einer Vielzahl von Template-Switching-Oligonukleotiden in die Partition und Barcodieren der Vielzahl von Zielnukleinsäuren durch Verlängern der Vielzahl von Barcode-Molekülen unter Verwendung der Zielnukleinsäuren und der Vielzahl von Template-Switching-Oligonukleotiden als Matrizen, um barcodierte Nukleinsäuren zu erzeugen; und/oder
(3) Einbringen einer Vielzahl von Verlängerungsprimern in die Partition und Barcodieren der Zielnukleinsäuren durch Verlängern der Vielzahl von Verlängerungsprimern unter Verwendung der Zielnukleinsäuren als Matrizen und der Vielzahl von Barcode-Molekülen als Template-Switching-Oligonukleotide, um barcodierte Nukleinsäuren zu erzeugen.

8. Verfahren nach einem der Ansprüche 1-7,
(1) wobei die Kopplungsgruppe des Kopplungsreagenzes in der Lage ist, eine kovalente Bindung mit der Oberfläche der Vielzahl von Zellen zu bilden,
(2) wobei das Kopplungsreagenz und/oder das Indexierungslabel ferner eine hydrophile Gruppe aufweist,
(3) wobei die hydrophilen Gruppen PEG beinhalten,
(4) wobei das Kopplungsreagenz NHS-PEG4-TCO ist; und/oder
(5) wobei die Kopplungsgruppe des Kopplungsreagenzes in der Lage ist, eine kovalente Bindung mit -NH₂ auf der Oberfläche der Vielzahl von Zellen zu bilden.

9. Verfahren nach einem der Ansprüche 1-8, wobei mindestens zwei Zellen aus derselben Probe mit identischen probenspezifischen Indexierungssequenzen markiert sind.

10. Verfahren nach einem der Ansprüche 1-9,
(1) wobei jedes der Indexierungslabels ein 5'-NHS-PEG5-Tz-modifiziertes Oligonukleotid ist,
(2) wobei jedes der Indexierungslabels ferner eine PCR-Handle-Sequenz, einen UMI, eine Capture-Sequenz oder eine Kombination davon aufweist, optional wobei die Capture-Sequenz eine Poly(dA)-Sequenz beinhaltet; und/oder
(3) wobei die Indexierungslabels einzelsträngige DNA sind.

11. Verfahren nach einem der Ansprüche 1-10, wobei eine der ersten reaktiven Gruppe und der zweiten reaktiven Gruppe eine Tetrazin-(Tz)-Gruppe aufweist und die andere eine trans-Cycloocten-(TCO)-Gruppe aufweist.

12. Verfahren nach einem der Ansprüche 1-11,
(1) wobei die Vielzahl von Proben mindestens 12 Proben aufweist, optional wobei die Proben klinische Proben, Umweltproben, biologische Proben oder eine Kombination davon sind; und/oder
(2) wobei die Vielzahl von Zellen prokaryotische Zellen, eukaryotische Zellen oder eine Kombination davon beinhaltet.

13. Verfahren nach einem der Ansprüche 1-12, das das Waschen von Folgendem aufweist:
(1) der Vielzahl von Zellen vor Schritt (a),
(2) der Vielzahl von Zellen, um ungebundenes Kopplungsreagenz nach Schritt (b) und vor Schritt (c) zu entfernen; und/oder
(3) den mit den probenspezifischen Indexierungslabels assoziierten Zellen, um ungebundene probenspezifische Indexierungslabels nach Schritt (c) zu entfernen; und/oder Resuspendieren der Zellen in einer wässrigen Lösung.

## Revendications

1. Procédé d'indexation d'une pluralité d'échantillons, comprenant :
(a) la fourniture d'une pluralité d'échantillons, dans lequel chacun de la pluralité d'échantillons comprend une pluralité de cellules, dans lequel la pluralité de cellules comprend des cellules vivantes et est dérivée de la séparation de cellules ;
(b) pour chacun de la pluralité d'échantillons, la mise en contact d'un agent de couplage avec l'échantillon, dans lequel l'agent de couplage comprend un groupe de couplage et un premier groupe réactif, associant ainsi l'agent de couplage à la surface de la pluralité de cellules ; et
(c) pour chacun de la pluralité d'échantillons, la mise en contact d'une pluralité de marqueurs d'indexation comprenant chacune une séquence d'indexation spécifique à l'échantillon identique et un second groupe réactif capable de former une liaison covalente avec le premier groupe réactif de l'agent de couplage dans une réaction de Diels-Alder à demande électronique inverses (IEDDA), générant ainsi une pluralité de cellules associées chacune au marqueur d'indexation spécifique à l'échantillon, dans lequel la séquence d'indexation spécifique à l'échantillon pour chaque échantillon est différente des autres échantillons.

2. Procédé d'analyse d'acides nucléiques, comprenant :
(a) la fourniture d'une pluralité d'échantillons, dans lequel chacun de la pluralité d'échantillons comprend une pluralité de cellules, dans lequel la pluralité de cellules comprend des cellules vivantes et est dérivée de la séparation de cellules ;
(b) pour chacun de la pluralité d'échantillons, la mise en contact d'un agent de couplage avec l'échantillon, dans lequel l'agent de couplage comprend un groupe de couplage et un premier groupe réactif, associant ainsi l'agent de couplage à la surface de la pluralité de cellules ;
(c) pour chacun de la pluralité d'échantillons, la mise en contact d'une pluralité de marqueurs d'indexation comprenant chacune une séquence d'indexation spécifique à l'échantillon identique et un second groupe réactif capable de former une liaison covalente avec le premier groupe réactif de l'agent de couplage dans une réaction de Diels-Alder à demande électronique inverse (IEDDA), générant ainsi une pluralité de cellules associées chacune au marqueur d'indexation spécifique à l'échantillon, dans lequel la séquence d'indexation spécifique à l'échantillon pour chaque échantillon est différente des autres échantillons ;
(d) le regroupement de la pluralité de cellules associées aux marqueurs d'indexation spécifiques à l'échantillon de la pluralité d'échantillons pour former un échantillon regroupé avec une pluralité de cellules regroupées ;
(e) le partitionnement de la pluralité de cellules regroupées en une pluralité de partitions, de sorte qu'au moins 25 % de la pluralité de partitions comprennent chacune une seule cellule de la pluralité de cellules regroupées ;
(f) l'analyse des acides nucléiques cibles associés à la cellule unique, dans lequel les acides nucléiques cibles comprennent les marqueurs d'indexation associés à la cellule unique, et
(g) la détermination de l'origine d'échantillon d'une cellule unique sur la base de la séquence d'indexation spécifique à l'échantillon associée à la cellule unique.

3. Procédé selon la revendication 2, dans lequel au moins 75 % de la pluralité de partitions comprennent chacune une cellule unique de la pluralité de cellules regroupées, et/ou dans lequel la pluralité de partitions comprend des gouttelettes ou des micro-puits.

4. Procédé selon l'une quelconque des revendications 2 à 3, dans lequel la détermination de l'origine d'échantillon de cellule unique comprend une dénaturation à haute température, un tri de fragments, ou une combinaison de ceux-ci.

5. Procédé selon l'une quelconque des revendications 2 à 4, dans lequel les acides nucléiques cibles comprennent :
(1) des acides nucléiques cellulaires, des acides nucléiques viraux, des acides nucléiques bactériens, des acides nucléiques mitochondriaux, des acides nucléiques synthétiques, ou un produit d'amplification de ceux-ci, ou une combinaison de ceux-ci ; et/ou
(2) de l'acide désoxyribonucléique (ADN), de l'acide ribonucléique (ARN) ou une combinaison de ceux-ci, et/ou
(3) de l'acide ribonucléique messager poly-adénylé (ARNm) de la cellule unique.

6. Procédé selon l'une quelconque des revendications 2 à 5, dans lequel l'analyse des acides nucléiques cibles comprend un codage à barres dans la pluralité de partitions comprenant une cellule unique, à l'aide d'une pluralité de molécules de code-barres dans une partition unique, pour générer des acides nucléiques cibles à code-barres, éventuellement dans lequel :
(1) le codage à barres des acides nucléiques cibles comprend :
(a) le codage à barres (i) des marqueurs d'indexation associés à la cellule unique et (ii) des ARNm des cellules individuelles pour générer (i-a) un marqueur d'indexation à code-barres et (ii-a) des ADNc à code-barres, et/ou
(b) une réaction de transcription inverse, et les acides nucléiques ciblés à code-barres comprennent de l'acide désoxyribonucléique complémentaire (ADNc) ;
(2) une molécule de code-barres de la pluralité de molécules de code-barres comprend une séquence de code-barres de cellule, une séquence de marquage moléculaire, une séquence d'amorce, un site de liaison d'amorce, un oligonucléotide de commutation de matrice, ou une combinaison de ceux-ci, éventuellement en outre dans lequel :
(a) les séquences de marquage moléculaire comprennent des identificateurs de molécules uniques (UMI),
(b) la séquence de marquage moléculaire a une longueur de 2 à 40 nucléotides, et/ou
(c) la séquence d'amorce est une séquence d'amorce de séquençage, éventuellement en outre dans lequel la séquence d'amorce de séquençage est une séquence de lecture 1, une séquence de lecture 2 ou une partie de celles-ci ;
(3) les molécules de code-barres de la pluralité de molécules de code-barres dans une seule partition comprennent une séquence de code-barres de cellule identique et une séquence de marqueurs moléculaires différente ; et/ou
(4) dans chacune de la pluralité de partitions comprenant une seule cellule, la pluralité de molécules de code-barres sont attachées à, attachées de manière réversible à, attachées de manière covalente ou irréversiblement attachées à une bille, éventuellement en outre dans lequel :
(a) le code-barres dans une partition unique comprend le partitionnement de la perle en une seule partition,
(b) le partitionnement de la pluralité de cellules regroupées en une pluralité de partitions comprend le co-partitionnement des cellules regroupées et de la perle en partitions simples,
(c) la bille est une bille solide, et/ou
(d) la bille est une bille magnétique ou une bille de polymère.

7. Procédé selon l'une quelconque des revendications 2 à 6, dans lequel l'analyse des acides nucléiques cibles comprend :
(1) le séquençage des acides nucléiques cibles, des produits d'amplification de ceux-ci, ou d'une partie des acides nucléiques cibles ou des produits d'amplification de ceux-ci ;
(2) l'introduction d'une pluralité d'oligonucléotides de commutation de matrice dans la partition et le codage à barres de la pluralité d'acides nucléiques cibles en étendant la pluralité de molécules de code-barres à l'aide des acides nucléiques cibles et de la pluralité d'oligonucléotides de commutation de matrice comme modèles pour générer des acides nucléiques à code-barres ; et/ou
(3) l'introduction d'une pluralité d'amorces d'extension dans la partition et le codage à barres des acides nucléiques cibles en étendant la pluralité d'amorces d'extension à l'aide des acides nucléiques cibles comme matrices et de la pluralité de molécules de code-barres comme oligonucléotides de commutation de matrice pour générer des acides nucléiques à code-barres.

8. Procédé selon l'une quelconque des revendications 1 à 7,
(1) dans lequel le groupe de couplage de l'agent de couplage est capable de former une liaison covalente avec la surface de la pluralité de cellules,
(2) dans lequel l'agent de couplage et/ou le marqueur d'indexation comprend en outre un groupe hydrophile,
(3) dans lequel les groupes hydrophiles comprennent du PEG,
(4) dans lequel l'agent de couplage est NHS-PEG4-TCO, et/ou
(5) dans lequel le groupe de couplage de l'agent de couplage est capable de former une liaison covalente avec -NH₂ à la surface de la pluralité de cellules.

9. Procédé selon l'une quelconque des revendications 1 à 8, dans lequel au moins deux cellules d'un même échantillon sont marquées avec des séquences d'indexation spécifiques à l'échantillon identiques.

10. Procédé selon l'une quelconque des revendications 1 à 9,
(1) dans lequel chacun des marqueurs d'indexation est un oligonucléotide modifié 5'NHS-PEG5-Tz,
(2) dans lequel chacun des marqueurs d'indexation comprend en outre une séquence de manipulation PCR, un UMI, une séquence de capture, ou une combinaison de celles-ci, éventuellement dans lequel la séquence de capture comprend une séquence poly(dA), et/ou
(3) dans lequel les marqueurs d'indexation sont de l'ADN simple brin.

11. Procédé selon l'une quelconque des revendications 1 à 10, dans lequel l'un du premier groupe réactif et du second groupe réactif comprend un groupe tétrazine (Tz) et l'autre comprend un groupe transcyclooctène (TCO).

12. Procédé selon l'une quelconque des revendications 1 à 11,
(1) dans lequel la pluralité d'échantillons comprend au moins 12 échantillons, éventuellement dans lequel les échantillons sont des échantillons cliniques, des échantillons environnementaux, des échantillons biologiques, ou une combinaison de ceux-ci ; et/ou
(2) dans lequel la pluralité de cellules comprend des cellules procaryotes, des cellules eucaryotes ou une combinaison de celles-ci.

13. Procédé selon l'une quelconque des revendications 1 à 12, comprenant le lavage
(1) de la pluralité de cellules avant l'étape (a),
(2) de la pluralité de cellules pour éliminer l'agent de couplage non lié après l'étape (b) et avant l'étape (c), et/ou
(3) des cellules associées aux marqueurs d'indexation spécifique à l'échantillon pour retirer les marqueurs d'indexation spécifique à l'échantillon non reliées après l'étape (c) ; et/ou de la remise en suspension des cellules dans une solution aqueuse.
